# EUROPEAN PATENT SPECIFICATION

(11) **EP 3 416 562 B1**
(45) Date of publication and mention of the grant of the patent: **23.12.2020**
(21) Application number: 16890359.9
(22) Date of filing: 10.11.2016
(51) Int. Cl.: A61B 6/03, A61B 6/02, A61B 6/04, A61B 6/00, G06T 3/40, G06T 5/50, H04N 5/232, G16H 15/00, G16H 30/00, A61B 90/00

(54) **SYSTEM AND METHOD FOR MEDICAL IMAGING**
SYSTEM UND VERFAHREN ZUR MEDIZINISCHEN BILDGEBUNG
SYSTÈME ET PROCÉDÉ D'IMAGERIE MÉDICALE

(30) Priority: 18.02.2016 CN 201610091173; 04.03.2016 CN 201610124014; 17.03.2016 CN 201610151647; 01.07.2016 US 201615201363
(43) Date of publication of application: 26.12.2018
(73) Proprietor: Shanghai United Imaging Healthcare Co., Ltd., Shanghai 201807 (CN)
(72) Inventor: MA, Runxia, Shanghai 201807 (CN); WANG, Lei, Shanghai 201807 (CN)
(74) Representative: Boult Wade Tennant LLP
(86) International application number: PCT/CN2016/105361
(87) International publication number: WO 2017/140133

(56) References cited:
- CN-A- 103 800 027
- CN-A- 104 217 447
- CN-A- 104 899 194
- CN-A- 105 740 214
- CN-A- 105 761 293
- CN-A- 105 796 122
- US-A1- 2005 171 423
- US-A1- 2008 221 520
- US-A1- 2012 029 341
- US-A1- 2015 112 183
- US-A1- 2015 223 771
- US-A1- 2015 363 948
- US-A1- 2015 363 948
- US-B1- 7 283 857

## Description

### TECHNICAL FIELD

The present disclosure generally relates to imaging, and more particularly, a system and method for operation controlling and data processing in imaging.

### BACKGROUND

Imaging methods including PET (Positron Emission Tomography), CT (Computed Tomography) and MRI (Magnetic Resonance Imaging) have been widely used in medical diagnosis. Merely by way of example, PET is a specialized radiology procedure that may generate images of functional processes in a target organ or tissue of a body. A biologically active molecule carrying a radioactive tracer is first introduced to a patient's body. The PET system then detects gamma rays emitted by the tracer, and an image indicating the tracer concentration distribution within the target organ or tissue of the body may be obtained based on the detected signals. The PET system may include a plurality of components. During a PET process, a plurality of processing parameters need to be controlled. There is a need for a system and method to control the components and processing parameters.
US 2015223771 A1 discloses a tomography apparatus and method of displaying a tomography image.
US 2012029341 A1 discloses a method and apparatus for acquiring overlapped medical image slices.
US 2015363948 A1 discloses a medical imaging system for reconstructing quantitative dynamic nuclear medicine images of a subject.
US 2008221520 A1 discloses a positioning system for percutaneous interventions.
US 2015112183 A1 discloses a MR Spectroscopy system and method.

### SUMMARY

In a first aspect of the present disclosure, a method for imaging according to claim 1 is provided.

In some embodiments, a template may be generated. The template may include at least a section identified by an index. Information regarding the subject may be obtained. The information may be added into the section according to the index.

In some embodiments, the template may include a HTML-formatted template.

In some embodiments, a color image-formatted file may be obtained. The color image-formatted file may be mapped with a grayscale image-formatted file. The grayscale image-formatted file may be converted into the one-dimensional data set.

In some embodiments, an interruption of the table positions may be detected according to the scanning protocol. Data acquired from the scan of the subject corresponding to the interruption may be deleted. The scanning protocol may be updated. A supplemental scanning may be performed from the interrupted table position based on the updated scanning protocol.

In some embodiments, the scanning protocol may include the number of the table positions and an order of the table positions.

In some embodiments, the number of the table positions may be at least one.

In some embodiments, a status of each one of the table positions may be detected. The interrupted table position may be determined based on the status of the table positions. The data acquired from the interrupted table position may be deleted.

In some embodiments, instructions relating to updating the scanning protocol may be obtained.

In some embodiments, the interrupted table position may be marked. The supplemental scanning may be performed from the marked table position.

In some embodiments, the acquired data may be segmented based on a segmenting mode. In some embodiments, the segmenting mode may include a time-based mode or a quantity-based mode. In some embodiments, in the time-based mode, the acquired data may be segmented based on acquisition time. In some embodiments, in the quantity-based mode, the acquired data may be segmented based on acquisition quantity of the acquired data.

In some embodiments, the data may be segmented based on a coincidence event curve. A data section may be generated based on the segmented data. The image may be reconstructed based on the data section.

In some embodiments, the data section may include a plurality of frames.

In some embodiments, a threshold may be set. A start value and an end value of the data may be set. A difference between the start value and the end value may be calculated. An alert may be provided when the difference is less than the threshold.

In some embodiments, a first plurality of data records including a first field in a first storage hierarchy may be detected. A second plurality of data records including a second field in a second storage hierarchy may be detected based on at least a first foreign key, the first foreign key including an identifier of the first plurality of data records. A third plurality of data records in a third storage hierarchy may be detected based on at least a second foreign key, the second foreign key including an identifier of the second plurality of data records. A route of a spare data file to be deleted may be acquired from the third plurality of data records. The spare data file may be deleted based on the acquired route.

In some embodiments, a scanning parameter may be set. In some embodiments, an operation may be determined. A notification corresponding to the operation may be provided to an operator. A response relating to the notification may be received from the operator. The operation may be performed based on the notification or the response.

In some embodiments, an enable signal corresponding to the operation may be provided.

In some embodiments, the notification may include an action relating to an operational component.

In some embodiments, the action may include long pressing, short pressing, or a combination of long pressing and short pressing.

In some embodiments, image information of the reconstructed image may be acquired. A reference image may be acquired. A reference line may be generated based on the reference image. The reconstructed image may be coupled with the reference line. A correlation between the reconstructed image and the reference image may be established based on the image information, the reference image, or the reference line.

In some embodiments, the image information may include image thickness, spacing, quality, shape of the reference line, orientation of the reference line, and image format.

In some embodiments, the reference image may include a PET image or a SPECT image.

In some embodiments, the scanning parameter and the acquired data may be separated. The scanning parameter may be stored in a DCM-formatted file. The acquired data may be stored in a binary-formatted file.

In a second aspect of the present disclosure, an imaging system according to claim 14 is provided.

In some embodiments, the system may further include a data deleting module. The operation control module may detect an interruption of the table positions according to the scanning protocol. The data deleting module may delete data acquired from the scan of the subject corresponding to the interruption. The operation control module may update the scanning protocol and perform a supplemental scanning from the interrupted table position based on the updated scanning protocol.

In some embodiments, the scanning protocol may include the number of the table positions and an order of the table positions.

In some embodiments, the number of the table positions may be at least one.

In some embodiments, the data deleting module may detect a status of each one of the table positions. The data deleting module may further determine the interrupted table position based on the status of the table position table. The data acquired from the interrupted table may be deleted.

In some embodiments, the data deleting module may receive instructions relating to updating the scanning protocol.

In some embodiments, the reconstruction module may include a segmenting unit. The segmenting unit may segment the acquired data based on a segmenting mode.

In some embodiments, the reconstruction module may include a segmenting unit configured to segment the acquired data based on a segmenting mode.

In some embodiments, the segmenting mode may include a time-based mode or a quantity-based mode.

In some embodiments, the reconstruction module may further include a coincidence event unit configured to generate a coincidence event curve, wherein the data is segmented based on the coincidence event curve.

In some embodiments, a data section may be generated based on the segmented data. In some embodiments, the data section may include a plurality of frames.

In some embodiments, the data segmenting unit may set a threshold; set a start value and an end value of the data; calculate a difference between the start value and the end value; and provide an alert when the difference is less than the threshold.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities and combinations set forth in the detailed examples discussed below.

### BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1 is a block diagram illustrating an imaging system according to some embodiments of the present disclosure;
FIG. 2 is a flowchart illustrating a process for processing signals according to some embodiments of the present disclosure;
FIG. 3 is a block diagram illustrating an architecture of a control engine according to some embodiments of the present disclosure;
FIG. 4 is a block diagram illustrating an architecture of a processing engine according to some embodiments of the present disclosure;
FIG. 5 is a block diagram illustrating an architecture of an operation control module according to some embodiments of the present disclosure;
FIG. 6 is a flowchart illustrating a process for operation control according to some embodiments of the present disclosure;
FIG. 7-A through FIG. 7-E provide an exemplary process for controlling operations according to some embodiments of the present disclosure;
FIG. 8 is a flowchart illustrating a process for acquiring/storing signals according to some embodiments of the present disclosure;
FIG. 9-A illustrates an exemplary process for deleting data according to some embodiments of the present disclosure;
FIG. 9-B illustrates an exemplary storage architecture according to some embodiments of the present disclosure;
FIG. 10 illustrates an exemplary process for controlling a scanning according to some embodiments of the present disclosure;
FIG. 11 is a block diagram illustrating an architecture of a reconstruction module according to some embodiments of the present disclosure;
FIG. 12 is a flowchart illustrating a process for reconstructing an image according to some embodiments of the present disclosure;
FIG. 13-A and FIG. 13-B illustrate an exemplary interface according to some embodiments of the present disclosure;
FIG. 14-A illustrates an exemplary process for reconstructing an image according to some embodiments of the present disclosure;
FIG. 14-B illustrates am exemplary interface according to some embodiments of the present disclosure;
FIG. 15 is a block diagram illustrating an architecture of a report generation module according to some embodiments of the present disclosure;
FIG. 16 is a flowchart illustrating a process for generating a report according to some embodiments of the present disclosure;
FIG. 17 illustrates an exemplary report according to some embodiments of the present disclosure;
FIG. 18 is a block diagram illustrating an architecture of a report generation module according to some embodiments of the present disclosure;
FIG. 19 is a flowchart illustrating a process for generating a report according to some embodiments of the present disclosure; and
FIG. 20 illustrates an exemplary interface according to some embodiments of the present disclosure.

### DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well known methods, procedures, systems, components, and/or circuitry have been described at a relatively high-level, without detail, in order to avoid unnecessarily obscuring aspects of the present disclosure. Various modifications to the disclosed embodiments will be readily apparent to those skilled in the art, and the general principles defined herein may be applied to other embodiments and applications. Thus, the present disclosure is not limited to the embodiments shown.

It will be understood that the term "system," "unit," "module," and/or "block" used herein are one method to distinguish different components, elements, parts, section or assembly of different level in ascending order. However, the terms may be displaced by other expression if they may achieve the same purpose.

It will be understood that when a unit, module or block is referred to as being "on," "connected to" or "coupled to" another unit, module, or block, it may be directly on, connected or coupled to the other unit, module, or block, or intervening unit, module, or block may be present, unless the context clearly indicates otherwise. As used herein, the term "and/or" includes any and all combinations of one or more of the associated listed items.

The terminology used herein is for the purposes of describing particular examples and embodiments only, and is not intended to be limiting. As used herein, the singular forms "a," "an," and "the" may be intended to include the plural forms as well, unless the context clearly indicates otherwise. It will be further understood that the terms "include," and/or "comprise," when used in this disclosure, specify the presence of integers, devices, behaviors, stated features, steps, elements, operations, and/or components, but do not exclude the presence or addition of one or more other integers, devices, behaviors, features, steps, elements, operations, components, and/or groups thereof.

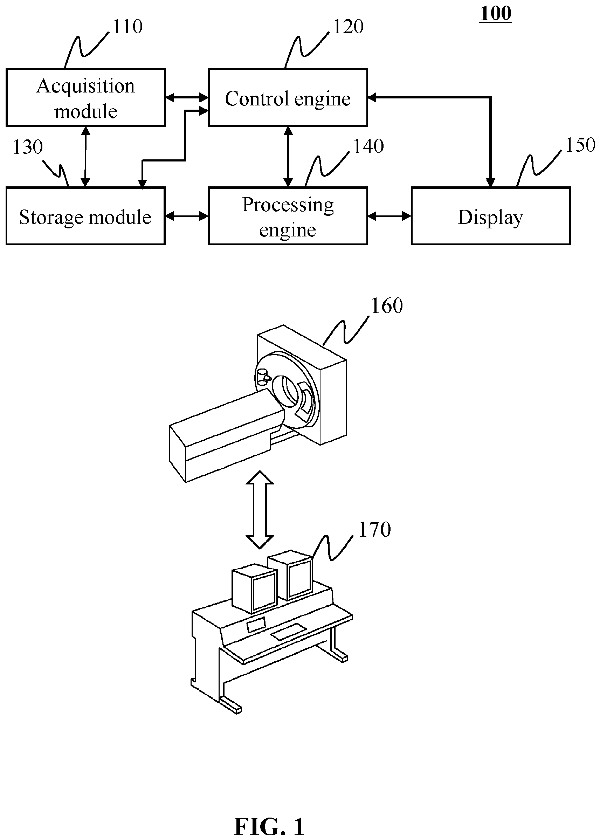

FIG. 1 is a block diagram of an imaging system 100 according to some embodiments of the present disclosure. It should be noted that the imaging system 100 described below is merely provided for illustration purposes, and not intended to limit the scope of the present disclosure. The radiation used herein may include a particle ray, a photon ray, or the like, or any combination thereof. The particle ray may include neutron, proton, electron, µ-meson, heavy ion, or the like, or any combination thereof. The photon beam may include X-ray, γ-ray, ultraviolet, laser, or the like, or any combination thereof. The imaging system may find its applications in different fields such as, for example, medicine or industry. Merely by way of example, the imaging system may be a positron emission tomography (PET) system, a single photon emission computed tomography (SPECT) system, a computed tomography (CT) system, a digital radiography (DR) system, a multi-modality system, or the like, or any combination thereof. Exemplary multi-modality system may include a computed tomography-positron emission tomography (CT-PET) system, a positron emission tomography-magnetic resonance imaging (PET-MRI) system, a computed tomography-positron emission tomography-magnetic resonance imaging (CT-PET-MRI) system, etc. As another example, the system may be used in internal inspection of components including, e.g., flaw detection, security scanning, failure analysis, metrology, assembly analysis, void analysis, wall thickness analysis, or the like, or any combination thereof.

As illustrated in FIG. 1, the imaging system 100 may include an acquisition module 110, a control engine 120, a storage module 130, a processing engine 140, and a display 150.

The acquisition module 110 may be used to detect radiation rays in the imaging system. Merely by way of example, the radiation rays may take the form of line of response (LOR) in a PET system. Detection of the LORs may be performed by the acquisition module 110 by way of counting values of coincidence from annihilation of positrons. As another example, the radiation rays may be X-ray beams passing through an object (e.g., a patient) in a CT system. The intensity of an X-ray beam passing through the object that lies between the X-ray source and a detector (not shown) may be attenuated, and further evaluated by the acquisition module 110. In some embodiments, the ROM may store programs for imaging of various types of nuclear medicine diagnosis. Exemplary types of nuclear medicine diagnosis may include PET, SPECT, CT, MRI, or the like, or a combination thereof. It should also be noted here that the "line of response" or "LOR" used here may be representative of a radiation ray, and not intended to limit the scope of the present disclosure. The radiation ray used herein may include a particle ray, a photon ray, or the like, or any combination thereof. The particle ray may include neutron, proton, electron, µ-meson, heavy ion, or the like, or any combination thereof. For example, the radiation ray may represent the intensity of an X-ray beam passing through the subject in the case of a CT system. As another example, the radiation ray may represent the probability of a positron generated in the case of a PET system.

The acquisition module 110 may select data to be further processed from the original data. The acquisition module 110 may measure the number of hits on the detector and determine, for example, the line of response (LOR) in the case of PET, the projected X-rays that pass through a subject in the case of CT, etc. In some embodiments, the acquisition module 110 may be a coincidence counting circuit in a PET case. Specifically, when a subject (e.g., a patient, etc.) takes a radioactive drug, two gamma rays may be generated by the annihilation of a positron. The gamma rays may be detected or registered by two opposing detector units of the PET system. For example, a coincidence counting circuit may check the incidence of the gamma rays, and determine the registered event to be proper data when the gamma rays impinge on the detector (not shown) at the opposite sides of the patient at or around the same time. The coincidence counting circuit may be part of the acquisition module 110. In some embodiments, the acquisition module 110 may be designed to surround a subject to form a table type scanner 160 (e.g., a CT scanner).

The control engine 120 may control the acquisition module 110, the storage module 130, the processing engine 140, and the display 150. The control engine 120 may receive information from and send information to the acquisition module 110, the storage module 130, the processing engine 140, and/or the display 150. In some embodiments, the control engine 120 may control the operation of the acquisition module 110. Merely for example, the control engine 120 may control whether to acquire a signal, or the time when the next signal acquisition may occur. As another example, the control engine 120 may control which section of radiation rays may be processed during an iteration of the reconstruction. The control engine 120 may control the processing engine 140, for example, to select different algorithms to process the raw data of an image, to determine the iteration times of the iteration projection process, and/or the location of the radiation rays. In some embodiments, the control engine 120 may receive a real-time or a predetermined command from the display 150 provided by a user including, e.g., an imaging technician, or a doctor, and adjust the acquisition module 110, and/or the processing engine 140 to take images of a subject of interest according to the received command. In some embodiments, the control engine 120 may communicate with the other modules for exchanging information relating to the operation of the scanner or other parts of the imaging system 100.

The storage module 130 may store the acquired signals, the control parameters, the processed signals, or the like. In some embodiments, the storage module 130 may include a random access memory (RAM), a read only memory (ROM), for example, a hard disk, a floppy disk, a cloud storage, a magnetic tape, a compact disk, a removable storage, or the like, or a combination thereof. The removable storage may read from and/or write data to a removable storage unit in a certain manner. The storage device may also be other similar means for loading computer programs or other instructions into the computer or processor.

The processing engine 140 may be configured or used to process different kinds of information received from different units. In some embodiments, the processing engine 140 may process the signals acquired by the acquisition module 110, or stored in the storage module 130. In some embodiments, the processing engine 140 may generate images, reports including one or more images and/or other related information, or the like, or a combination thereof. In some embodiments, the processing engine 140 may process the information displayed in the display 150.

The display 150 may receive input and/or display output information. The display may include a liquid crystal display (LCD), a light emitting diode (LED)-based display, or any other flat panel display, or may use a cathode ray tube (CRT), a touch screen, or the like. A touch screen may include, e.g., a resistance touch screen, a capacity touch screen, a plasma touch screen, a vector pressure sensing touch screen, an infrared touch screen, or the like, or a combination thereof.

Further, while not shown, the imaging system 100 may be connected to a network (e.g., a telecommunications network, a local area network (LAN), a wireless network, a wide area network (WAN) such as the Internet, a peer-to-peer network, a cable network, etc.) for communication purposes.

For further understanding the present disclosure, several examples are given below, but the examples do not limit the scope of the present disclosure. For example, in some embodiments, the processing engine 140 may process signals received from the acquisition module 110 and generate one or more images based on these signals and deliver the images to the display 150. In some embodiments, the processing engine 140 may process data input by a user or an operator via the display 150 and transform the data into specific commands, and supply the commands to the control engine 120. The display 150 may receive input and/or display output information. The input and/or output information may include programs, software, algorithms, data, text, number, images, voice, or the like, or any combination thereof. For example, a user or an operator may input some initial parameters or conditions to initiate a scan. As another example, some information may be imported from an external resource, such as a floppy disk, a hard disk, a wireless terminal, or the like, or any combination thereof. In some embodiments, the control engine 120, the storage module 130, the processing engine 140, and/or the display 150 may be integrated into a console 170. Users may set parameters in scanning, control the imaging procedure, view the images produced through the console 170.

It should be noted that the above description of the imaging system 100 is merely provided for the purposes of illustration, and not intended to limit the scope of the present disclosure. For persons having ordinary skills in the art, multiple variations and modifications may be made under the teachings of the present disclosure. For example, the assembly and/or function of the imaging system 100 may be varied or changed according to specific implementation scenarios. Merely by way of example, some other components may be added into the imaging system 100, such as a patient positioning module, a gradient amplifier module, and other devices or modules. As another example, the storage module 130 is unnecessary and the engines or modules in the imaging system 100 may include an integrated storage unit respectively. Note that the imaging system may be a traditional or a single-modality medical system, or a multi-modality system including, e.g., a positron emission tomography-magnetic resonance imaging (PET-MRI) system, a single photon emission computed tomography-magnetic resonance imaging (SPECT-MRI) system, a remote medical MRI system, and others, etc. However, those variations and modifications do not depart from the scope of the present disclosure.

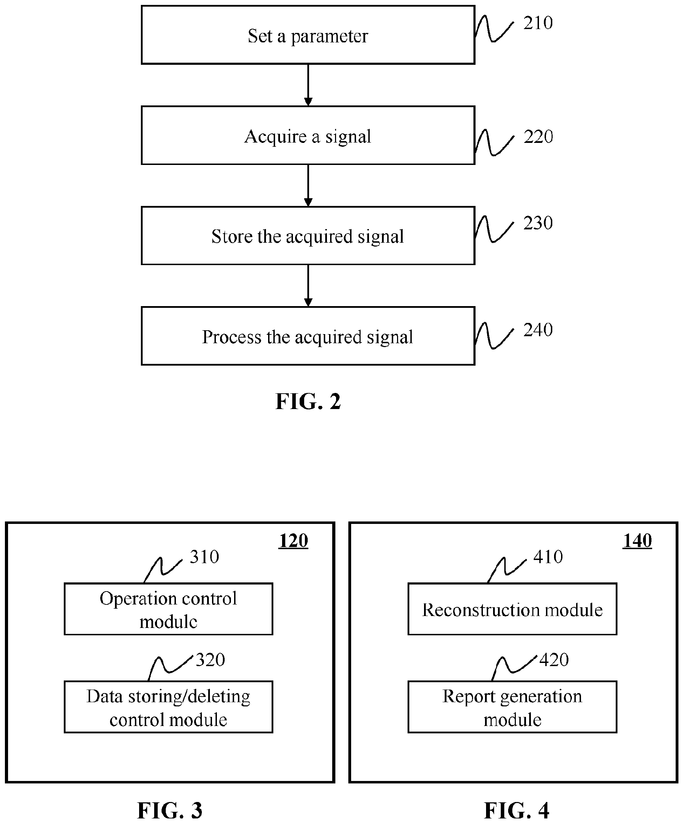

FIG. 2 is a flowchart of processing signals according to some embodiments of the present disclosure. In step 210, a parameter may be set. The parameter may be set by the control engine 120. In some embodiments, the parameter may include a parameter related to an acquisition process, a parameter related to a storing process, a processing parameter, a parameter related to a displaying process, or the like, or a combination thereof. Merely by way of example, the parameter may include current, voltage, a scanning protocol designed for one or more tissues to be imaged, diseases, and/or clinical scenarios, a workflow including a plurality of operations, sampling speed, sampling frequency, storage speed, storage volume management, image reconstruction method, or the like, or a combination thereof. In some embodiments, the parameter may be set via the console 170.

In step 220, a signal may be acquired. The signal may be a PET signal, a CT signal, a SPECT signal, a MRI signal, or the like, or a combination thereof. In some embodiments, the signal acquisition may be performed by the acquisition module 110. In some embodiments, the signal may be acquired from the storage module 130. In some embodiments, the signal may be loaded from an external device or via a user input. In step 230, the acquired signal may be stored. The acquired signal may be stored in the storage module 130 or any storage disclosed anywhere in the present disclosure. In some embodiments, step 220 and step 230 may be integrated into a single step in which the signal may be acquired and stored simultaneously or successively.

In step 240, the signal may be processed. The processing may be performed by the processing engine 140. During the processing, one or more processing parameters may be set. In some embodiments, the signal may be processed to reconstruct an image (e.g., a PET image, a CT image, a SPECT image, a MRI image, or the like). In some embodiments, the reconstructed image may be further processed and a report including the reconstructed image may be generated. In some embodiments, the reconstructed image and/or the generated report may be transmitted to a related device (e.g., a terminal, a database, or the like). In some embodiments, the reconstructed image and/or the generated report may be transmitted to a related device to be further processed (e.g., to be printed, to be displayed, or the like).

It should be noted that the above description is provided for the purposes of illustration, not intended to limit the scope of the present disclosure. For persons having ordinary skills in the art, multiple variations and modifications may be reduced to practice in the light of the present disclosure. However, those variations and modifications do not depart from the scope of the present disclosure. For example, step 230 is unnecessary, the acquired signal may be processed directly in step 240 without storing. As another example, the parameter may be set during any step of the whole process.

FIG. 3 is a block diagram illustrating an architecture of the control engine 120 according to some embodiments of the present disclosure. As illustrated in FIG. 3, the control engine 120 may include an operation control module 310 and a data storing/deleting control module 320. The operation control module 310 and the data storing/deleting control module 320 may be connected with each other via a wired or a wireless connection. As used herein, a module may have an independent processor, or use system shared processor(s). The processor(s) may perform functions according to instructions related to various modules.

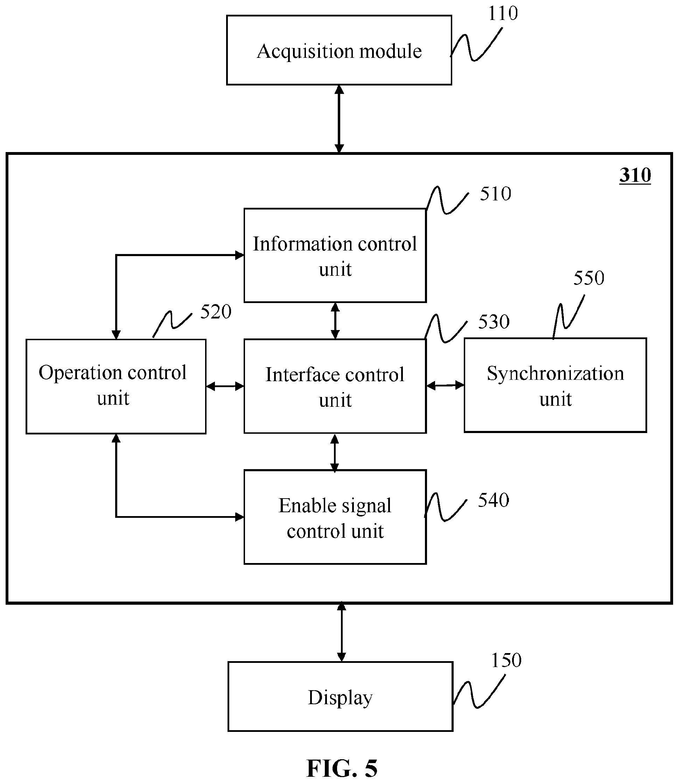

The operation control module 310 may be used to control one or more parameters related to operations performed by any module or unit in the system 100. In some embodiments, the operation may include selecting a scanning protocol, setting a scanning position, moving a table to a setting position, starting a scanning, completing a scanning, setting a processing parameter, selecting an algorithm for reconstructing an image, or the like, or a combination thereof. In some embodiments, the operation control module 310 may include one or more units (see details in FIG. 5).

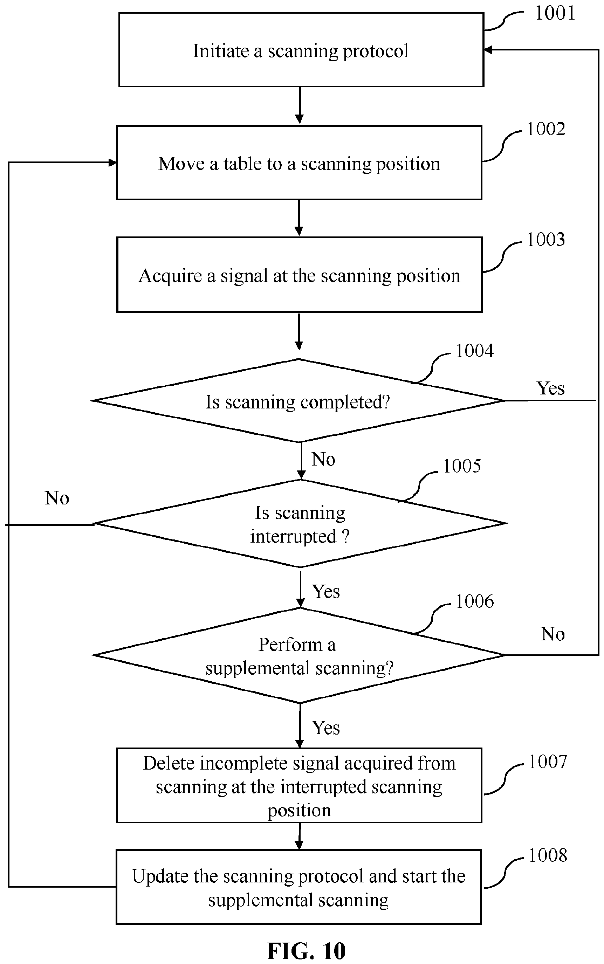

The data storing/deleting control module 320 may be used to control the storing or deleting of the acquired signals and/or any generated data or intermediate data (e.g., the reconstructed image, the generated report, or the like). In some embodiments, the acquired signals may be stored according to a predefined rule. For example, the acquired raw data and one or more acquisition parameters may be stored separately. As another example, the acquired raw data and data related to coincidence events may be stored separately. In some embodiments, a parameter related to a storing process may be adjusted during the storing, e.g., storing speed, storing volume, storage format, or the like, or a combination thereof. In some embodiments, the signals or data may be deleted automatically according to a predefined rule. For example, if the available volume of the storage module 130 is less than a preset threshold, data that satisfies a preset condition may be deleted for releasing storage volume. In some embodiments, the data storing/deleting module 130 may include a detection unit (not shown) used to detect data or signals to be deleted. In some embodiments, the data storing/deleting module 130 may include a determination unit (not shown) used to determine whether to delete data or signals according to a specific rule (for example, see, FIG. 10).

It should be noted that the above description is provided for the purposes of illustration, not intended to limit the scope of the present disclosure. For persons having ordinary skills in the art, multiple variations and modifications may be reduced to practice in the light of the present disclosure. However, those variations and modifications do not depart from the scope of the present disclosure. For example, a cache unit or a storage unit may be added to the control engine 120 used for storing an intermediate result or real time signal or information during the processes above mentioned.

FIG. 4 is a block diagram illustrating an architecture of the processing engine 140 according to some embodiments of the present disclosure. As illustrated in FIG. 4, the processing engine 140 includes a reconstruction module 410 and a report generation module. The reconstruction module 410 and the report generation module may be connected with each other via a wired or a wireless connection. As used herein, a module may have an independent processor, or use system shared processor(s). The processor(s) may perform functions according to instructions related to various modules.

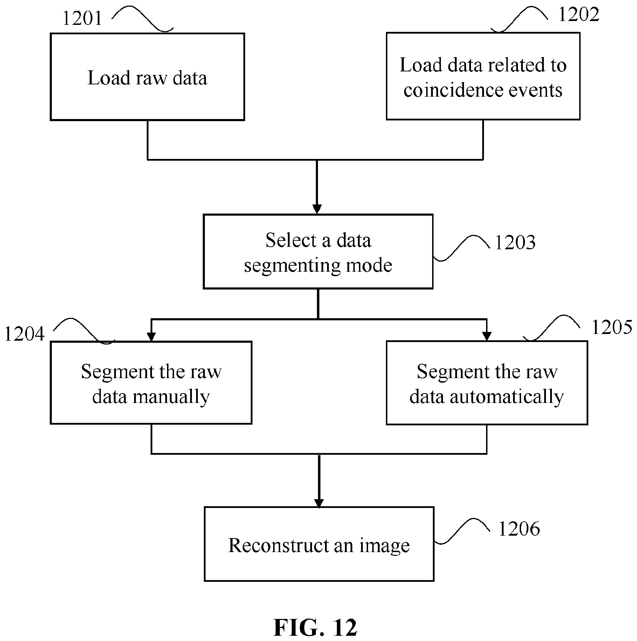

The reconstruction module 410 may be used to reconstruct an image based on the acquired signals. The image may include a PET image, a CT image, a MRI image, a SPECT image, or the like, or a combination thereof. For example, in a PET system, a PET image may be reconstructed based on one or more data sections incised by a data incision method (see details in FIGs. 12-14). As another example, in an MRI system, the reconstruction module 410 may spatially decode an MR signal that has been spatially encoded by the magnetic field(s). In some embodiments, the reconstruction module 410 may employ different kinds of imaging reconstruction techniques for the image reconstruction procedure. Exemplary image reconstruction techniques may include Fourier reconstruction, constrained image reconstruction, regularized image reconstruction in parallel MRI, or the like, or a variation thereof, or any combination thereof.

The report generation module 420 may generate a report including the reconstructed image, and/or some other related information. The related information may include basic information regarding a subject (e.g., age, gender, weight, height, health history, or the like), and/or examination information (e.g., scanning protocol, scanning time, reconstruction sequence, or the like). The format of the report may include HTML, ASP (Active Server Page), PHP (Hypertext Preprocessor), or the like. In some embodiments, the report generation module 420 may receive data or information from the acquisition module 110, the storage module 130, or the like, or a combination thereof. For example, one or more parameters related to an acquisition process may be received.

In some embodiments, the reconstructed image and/or the generated report may be further processed. For example, the reconstructed image and/or the generated report may be transmitted to the display 150, a database (not shown), an external device (e.g., a terminal), or the like, or a combination thereof. As another example, the reconstructed image and/or the generated report may be further processed. For example, a correlation among the image and/or some other related information (e.g., basic information regarding a subject) may be generated and provided to an external device (e.g., a display, a terminal, a printer, or the like).

It should be noted that the above description is provided for the purposes of illustration, not intended to limit the scope of the present disclosure. For persons having ordinary skills in the art, multiple variations and modifications may be reduced to practice in the light of the present disclosure. However, those variations and modifications do not depart from the scope of the present disclosure. For example, the processing engine 140 may include one or more storage modules (not shown) used for storing the reconstructed images and/or the generated reports. As another example, one or more additional components such as an interface block, a transmission block, etc., may be added into the processing engine 140.

FIG. 5 is a block diagram illustrating an architecture of the operation control module 310 according to some embodiments of the present disclosure. As shown in FIG. 5, the operation control module 310 may include an information control unit 510, an operation control unit 520, an interface control unit 530, an enable signal control unit 540, and a synchronization unit 550. The operation control module 310 may be connected with or otherwise communicate with the acquisition module 110 and the display 150 via a wired or wireless connection.

The information control unit 510 may control a process relating to information. Exemplary processes may include loading, editing, analyzing, separating, storing, managing, processing, updating, or the like, or a combination thereof. The information may include information relating to a subject (e.g., name, age, gender, height, weight, health history, or the like), environmental information (e.g., temperature, humidity, gas composition, air pressure, noise, or the like), a scanning parameter (e.g., scanning time, scanning position, scanning intensity, or the like), a processing parameter (e.g., reconstruction method, reconstruction sequence, or the like), system setting data (e.g., power-on setting), or the like, or a combination thereof. In some embodiments, the information control unit 510 may include one or more sub-units (not shown) used to control processes regarding different information mentioned above respectively. In some embodiments, the information control unit 510 may load information from the acquisition module 110, or may receive information from the operation control unit 520, or may receive a user input via the interface control unit 530.

The operation control unit 520 may control a parameter of an operation. The parameter may include a scanning protocol, a scanning position, the speed of a table moving relating to a scanning, the distance between any two table positions, or the like, or a combination thereof. In some embodiments, the parameter may be a default setting of the imaging system 100, or may be set by an operator (e.g., a doctor) based on the information relating to the subject and/or the environmental information, etc. Merely by way of example, different scanning protocols may be set for different subjects of different ages (e.g., the young, the old, or the like). In some embodiments, the operation control unit 520 may set or modify one or more operations. The operation may include starting the system, setting a scanning protocol, moving a table, starting a scanning, reconstructing an image, displaying information or an image, or the like, or a combination thereof. For example, a workflow including a plurality of operations may be set. As another example, a pre-set operation may be modified (e.g., a pre-set scanning protocol may be modified). As a further example, one or more operations in a workflow may be cancelled. As a still further example, a relationship between an operation and an operational component may be built. In the relationship, a specific operation may correspond to a specific action (e.g., long press, short press, click, double-click, or the like) regarding a specific operational component (e.g., a button, a handle, an icon, or the like). Merely by way of example, an operation "moving a table" may correspond to an action "long pressing" a button, in which the operational component is the "button." In some embodiments, the operation control unit 520 may detect an interruption of a scanning (see details in FIG. 10).

The enable signal control unit 540 may generate and/or provide an enable signal. In some embodiments, the enable signal control unit 540 may receive an operation instruction from the operation control unit 520, and may provide a corresponding enable signal that may relate to a specific action regarding a specific operational component. As used herein, the enable signal may be used to confirm that the corresponding operation (and also the corresponding action regarding the corresponding operational component) is valid, and the corresponding operation may be performed based on the enable signal. Merely by way of example, an operation instruction for an operation "moving a table" is received. As described above, a specific operation may correspond to a specific action regarding a specific operational component. In this case, the corresponding action may be "pressing" and the operational component may be "a button." After the instruction is received, an enable signal that corresponds to the operation (also the corresponding action regarding the operational component) may be provided. For instance, an enable signal corresponding to "pressing a button" may be provided. In some embodiments, the enable signal may be transmitted to the interface control unit 530 and may be used to determine whether a user instruction matches the enable signal. As used herein, a user instruction may refer to a user request for performing an action regarding an operational component (e.g., clicking an icon).

The interface control unit 530 may provide an interface for the information control unit 510, the operation control unit 520, the enable signal control unit 540, and the synchronization unit 550. In some embodiments, information interaction among the units may be implemented via the interface. A user may input or edit information via the interface. The system may provide a notification via the interface. In some embodiments, a user instruction may be received via the interface. As used herein, a user instruction may refer to a user request for performing an action regarding an operational component. The received user instruction may be transmitted to the information control unit 510, the operation control unit 520, and/or the enable signal control unit 540 to be further analyzed (e.g., the user instruction may be compared with a generated enable signal to determine whether it matches the enable signal). In some embodiments, the interface control unit 530 may communicate with one or more related devices (e.g., the acquisition module 110, the display 150, the storage module 130, or the like).

In some embodiments, the synchronization unit 550 may control a process for synchronizing information among the units in the operation control module 310. In some embodiments, the synchronization unit 550 may be connected with the interface unit 530 to communicate with the information control unit 510, the enable signal control unit 540, and the operation control unit 520. Merely by way of example, if information regarding a subject (e.g., medical history related information) is edited via the information control unit 510, the edited information may be updated to the operation control unit 520 synchronously. In some embodiments, the synchronization unit 550 may control a process for synchronizing information among the operation control module 310 and other modules or units in the system, e.g., the display 150, an external device (e.g., a printer), a terminal (e.g., a computer, a mobile phone, or the like), a storage device (e.g., a hard disk, a cloud storage, a removable storage, or the like), or the like, or a combination thereof. In some embodiments, a synchronization parameter may be set based on a default setting of the imaging system 100, or by an operator (e.g., a doctor). The synchronization parameter may include time interval of synchronization, synchronization speed, synchronization frequency, or the like, or a combination thereof.

It should be noted that the description above is provided for the purposes of illustration, not intended to limit the scope of the present disclosure. For persons having ordinary skills in the art, multiple variations and modifications may be reduced to practice in the light of the present disclosure. However, those variations and modifications do not depart from the scope of the present disclosure. For example, the operation control unit 520 and the information control unit 510 may be integrated in an independent unit configured for controlling both related information and operations. The independent unit may be connected with other units via a wired or a wireless connection. As another example, the units may be partially integrated in one or more independent units or share one or more sub-units.

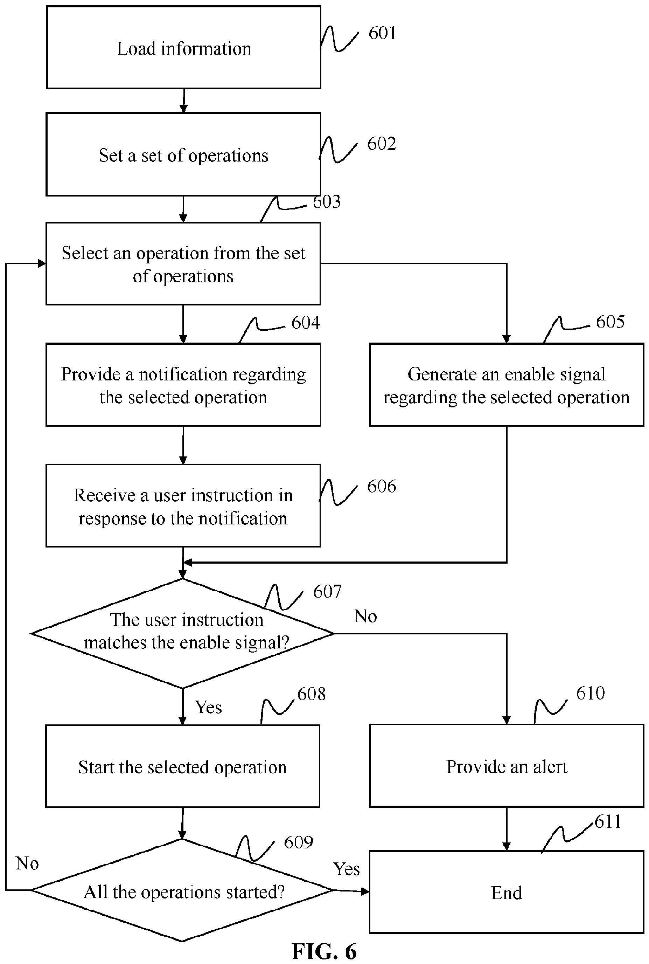

FIG. 6 illustrates an exemplary process for controlling operations according to some embodiments of the present disclosure. In step 601, information may be loaded. The information may include information related to a subject (e.g., age, gender, weight, height, health history, or the like), environmental information (e.g., temperature, humidity, gas composition, air pressure, noise, or the like), a scanning parameter (e.g., scanning time, scanning position, scanning intensity, or the like), system setting data (e.g., power-on setting), or the like, or a combination thereof.

The information may be loaded from the acquisition module 110, the storage module 130 or any storage disclosed anywhere in the present disclosure. The loading information may be performed by the information control unit 510. In some embodiments, the information may be input by the subject or an operator (e.g., a doctor). For example, a doctor may input a recommendation regarding a health examination (e.g., examining whether an abnormality occurs in an organ via a PET scanning).

In step 602, a set of operations may be set. The setting the set of operations may be performed by the operation control unit 520. The set of operations may include setting a scanning protocol, moving a table to a specific position, moving a table to a scanning position, setting a parameter related to the table(s) (e.g., horizontal position, vertical position, tilt angle, or the like), starting a scanning, completing a scanning, or the like, or a combination thereof. As used herein, a scanning protocol may refer to the number of table positions of the table, an order of the table positions, the number of scanning positions, scanning sequence, the start time and the end time of acquiring signals at each scanning positions, and/or any other related information. In some embodiments, the operations may be set based on a default setting of the imaging system 100, e.g., the system may determine a plurality of operations corresponding to different kinds of subjects (e.g., the young, the old, or the like). In some embodiments, the operations may be set by an operator (e.g., a doctor) based on the information loaded in step 601. For example, if the health history indicates that the subject suffered a hepatic problem, an operation corresponding to a scanning of the liver area may be set. As another example, the environmental information may be taken into consideration, for example, an environmental requirement for an operation may be aseptic, low noise level, or the like, or a combination thereof. As a further example, one or more scanning parameters may be loaded and used for setting the set of operations. In some embodiments, the set of operations may be set in an interactive manner. The interactive manner may include manual input, voice input, scanning a QR code, or the like, or a combination thereof.

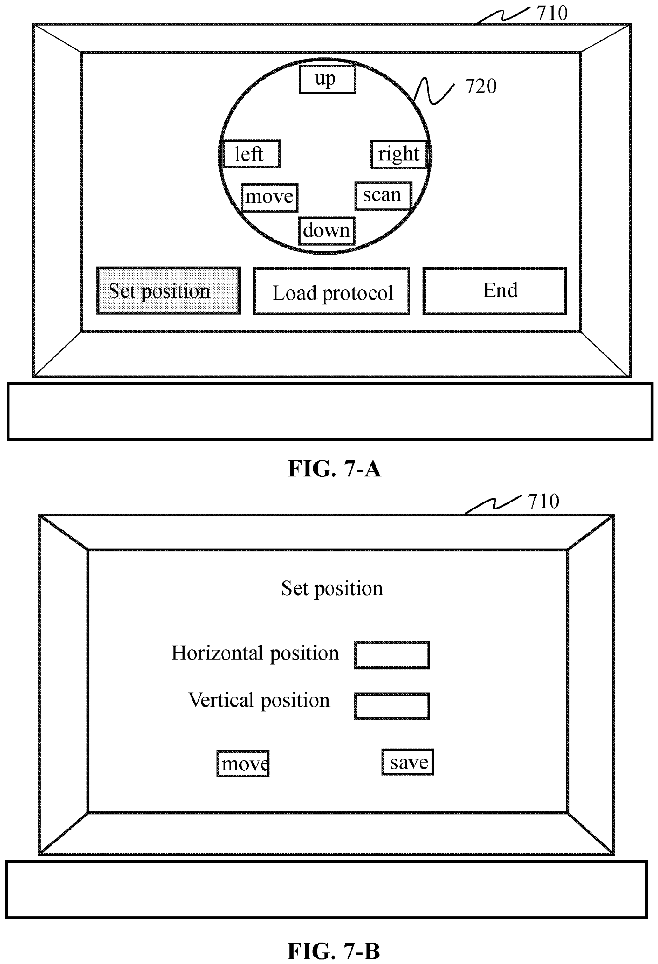

In some embodiments, the set of operations may be one single operation (e.g., starting a scanning), or a plurality of operations. In some embodiments, the plurality of operations may be set to be operated in a certain order (e.g., a workflow). In some embodiments, the workflow may include loading a scanning protocol first, moving a table to a scanning position and starting a scanning at a time point, or the like. The description regarding the workflow is merely provided for the purposes of illustration, and not intended to limit the scope of the present disclosure. In some embodiments, a relationship between an operation and a corresponding operational component may be built. For example, a scanning may be started by an operator pressing a button. The relationship or correlation between an action, an operational component, and the corresponding operation may be built based on a default setting of the imaging system 100, or may be customized by an operator. In some embodiments, the operational component may include an actual component (e.g., a button in a control box) and a virtual component (e.g., a virtual button on an interface) (see details in FIGs 7-A through 7-E).

In step 603, an operation may be selected from the set of operations. The selection may be performed by the operation control unit 520. The selection may be performed based on a default setting of the imaging system 100 (e.g., a preset workflow), or performed by an operator (e.g., a doctor). For example, an operator may manually select an operation according to the information loaded in step 601 (e.g., a recommendation provided by a doctor).

In step 604, a notification corresponding to the selected operation may be provided. The notification may be provided by the interface control unit 530. The notification may include a recommended action regarding an operational component (e.g., the recommended action may be long pressing, and the operational component may be a button) that may trigger the selected operation. The recommended action may include a touch-based manner, a non touch-based manner, or the like. As used herein, the touch-based manner may refer to directly touching an operational component. The non touch-based manner may refer to communicating with an operational component in a non touch way (e.g., voice, sensing, or the like). Merely by way of example, the recommended action may include "hold," "long press," "short press," "double-click," "click," or the like, or any combination thereof. As used herein, "hold" may refer to that a corresponding operation may last until the action stops, "short press" may refer to that a corresponding operation may start immediately after a short press, "double-click" or "click" may refer to that a corresponding operation may start after an action of double clicking or clicking an icon is detected or received. These exemplary recommended actions are merely provided for the purposes of illustration, and not intended to limit the scope of the present disclosure.

In some embodiments, the notification may include, for example, an audio notification, a haptic notification, a text notification, a picture notification, a video notification, or the like, or a combination thereof. In some embodiments, the notification may be synchronized among one or more devices relating to the system (e.g., the display 150, a display on the scanning gantry, a user interface shown on a terminal, or any device that may be used to show the notification).

In step 605, an enable signal regarding the operation selected in step 603 may be generated. The enable signal generation may be performed by the enable signal control unit 540. The enable signal may correspond to a specific action regarding a specific operational component, and may be used to confirm that the corresponding action is valid.

In step 606, the system may receive a user instruction in response to the notification provided in step 604. In some embodiments, the user instruction may be obtained from the interface control unit 530. The user instruction may be an instruction to perform a specific action regarding a specific operational component (e.g., an instruction indicating that an operator expects to press a button). In some embodiments, the user instruction may refer to an instruction indicating that the operator has performed a specific action regarding a specific operational component (e.g., an instruction indicating that the operator has pressed a button).

In step 607, the system may determine whether the user instruction matches the enable signal. As used herein, "match" may refer to that the action regarding the operational component corresponding to the user instruction is consistent with the action corresponding to the enable signal generated in step 605. Merely by way of example, if the operation selected in step 603 is "moving a table," and according to the relationship between the operations and the operational components, the action regarding the operational component that corresponds to the operation may be "short pressing a button." The enable signal generated in step 605 may be a signal indicating that if the button is short pressed the table may be moved. In step 607, if the user instruction is a user request for "short pressing the button," it may indicate that the user instruction matches the enable signal. Otherwise, if the user instruction is a user request for "long pressing the button," it may indicate that the user instruction does not match the enable signal. As another example, if the user instruction is a user request for "long pressing the button," the system may determine the user instruction is invalid.

In step 607, if the answer is "no," i.e., the user instruction does not match the enable signal, an alert may be provided in step 610 and the process may end in step 611. The alert may be provided in the format of, for example, text, audio, video, picture, a haptic effect, or the like, or a combination thereof. In some embodiments, the alert may be synchronized to a related device (e.g., a terminal). In some embodiments, the notification may be resent, a new user instruction may be received, and a new process may start from step 606 until the user instruction matches the enable signal. In some embodiments, an operation may be allowed to provide user instructions relating to an operation for a certain number of times; after a certain number of failed attempts, the workflow may be temporarily suspended or terminated. If the answer is "yes," i.e., the user instruction matches the enable signal, the selected operation may be started in step 608. The starting the selected operation may be performed by the operation control unit 520.

In step 609, the system may determine whether all the operations are performed. If the answer is "yes," the process may end in step 611. If the answer is "no," the process may return to step 603 to select another operation to be performed. In some embodiments, even though one or more operations are not performed, the process may end if an emergency (e.g., a system malfunction, the subject being examined has a situation that needs an immediate attention, etc.) or another situation (e.g., too many failed attempts by an operation, the duration of a set of operation in a specific case exceeds a threshold for some unusual reasons, etc.) occurs.

It should be noted that the flowchart described above is provided for the purposes of illustration, not intended to limit the scope of the present disclosure. For persons having ordinary skills in the art, multiple variations and modifications may be reduced to practice in the light of the present disclosure. However, those variations and modifications do not depart from the scope of the present disclosure. For example, step 601 may be unnecessary. As another example, the providing the notification (step 604) and the enable signal generation (step 605) may be performed simultaneously or successively.

FIGs. 7-A through 7-E illustrate an exemplary process for operation control according to some embodiments of the present disclosure. In this embodiment, an operation control may be with respect to a workflow including a plurality of operations. The workflow may include setting a table parameter, moving a table to a setting position, loading a scanning protocol, moving the table to a scanning position, starting the scanning, ending the scanning, or the like, or a combination thereof. Within a workflow, the table may be located at different table positions including, for example, a setting position, a scanning position, etc. Furthermore, as shown in Table 1, a relationship between the operations and the corresponding operational components may be established.

**Table 1 Relationship between operations and corresponding operational components**

| **Order** | **Operation** | **Operational component** |
|---|---|---|
| 1 | setting a table parameter | "set position" button in the central processor |
| 2 | moving a table to a setting position | "up," "down," "left," and "right" buttons in the virtual control box and the physical control box |
| 3 | loading a scanning protocol | "load protocol" button in the central processor |
| 4 | moving the table to a scanning position | "move" button in the virtual control box and the physical control box |
| 5 | starting the scanning | "scan" button in the virtual control box and the physical control box |
| 6 | ending the scanning | "end" button in the central processor |

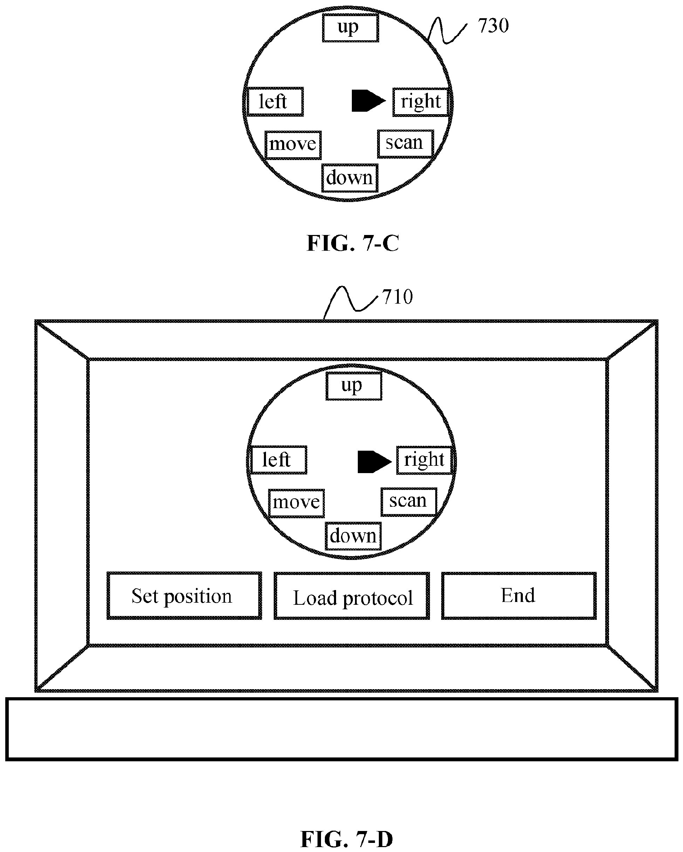

As shown in FIG. 7-A, an interface in a central processor 710 may be provided. The central processor 710 may be integrated in the console 170. A plurality of buttons including "set position," "load protocol," and "end" and a virtual control box 720 may be shown. The virtual control box 720 may include a plurality of buttons, e.g., "up," "down," "left," "right," "move," and "scan." The virtual control box 720 may correspond to a physical control box 730 illustrated in FIG. 7-C that includes a plurality of corresponding buttons, e.g., "up," "down," "left," "right," "move," and "scan."

In some embodiments, the central processor 710 and/or the console 170 may be located in a first room (e.g., a control room), while the physical control box 730 may be located in the first room, or implemented on a control station located in a second room (e.g., the examination room where images are taken, etc.). Furthermore, the physical control box 730 may connect with the central processor 710 via a wireless and/or a wired connection. User input (including, for example, user instructions, an action with respect to an operational component, etc.) may be received from either the central processor 710 (and/or the console 170), or the control station. In some embodiments, multiple operators may provide user input directed to a same imaging device from the central processor 710 (and/or the console 170) and from the control station. In some embodiments, an operation may proceed if both the user input from the central processor 710 (and/or the console 170) and the user input from the control station are consistent with each other and with the corresponding system setting in the form of, e.g., an enable signal. In some embodiments, an operation may proceed if user input from either the central processor 710 (and/or the console 170) or from the control station is consistent with the corresponding system setting in the form of, e.g., an enable signal. In some embodiments, if user input from the central processor 710 (and/or the console 170) is inconsistent with the user input from the control station, the operation may not proceed, and/or an alert is generated. In some embodiments, a type of user input may overwrite some user input. For instance, a user input of an emergency termination of an operation, regardless of whether it is received from the control station or from the central processor 710 (or the console 170), overwrites any other user input to initiate a normal operation (e.g., an operation relating to moving the table, performing a scan, etc.).

As illustrated in Table 1, operations may be conducted in an order based on the preset workflow. First, setting a table parameter may be initiated via the "set position" button. A notification corresponding to the operation "setting a table parameter" may be provided, e.g., the "set position" button may be lit or flashing, indicating a recommended action "pressing the button". Simultaneously or successively an enable signal corresponding to the operation "setting a table parameter" (also the action regarding the operational component "pressing the button") may be generated. If the "set position" button is pressed, the corresponding operation "setting a table parameter" may be started, and as shown in FIG. 7-B, a horizontal position and a vertical position may be set. Via the interface, the horizontal position and the vertical position may be provided by, e.g., an operator. The received horizontal position and the vertical position may be saved. When the operation "setting a table parameter" has been started, the corresponding notification may be closed, simultaneously or successively the enable signal may be turned off.

Refer back to Table 1, a next operation "moving a table to a setting position" may be selected. The corresponding operational components may include the buttons "up," "down," "left," "right," "move," and "scan" in the virtual control box 720 and/or the physical control box 730. A notification corresponding to the operation "moving a table to a setting position" may be provided, for example, the buttons "up," "down," "left," "right," "move," and "scan" in the virtual control box 720 and the physical control box 730 may be lit or flashing, indicating a recommended action "pressing the button" (e.g., see FIG. 7-C and FIG. 7-D). Simultaneously or successively one or more enable signals corresponding to the operation "moving a table to a setting position" (also the action(s) regarding the operational component(s) "pressing the button(s)") may be generated. Via one or more correct actions, the operation "moving a table to a setting position" may be started. When the operation "moving a table to a setting position" has been started, the corresponding notification may be closed, simultaneously or successively the enable signal may be turned off.

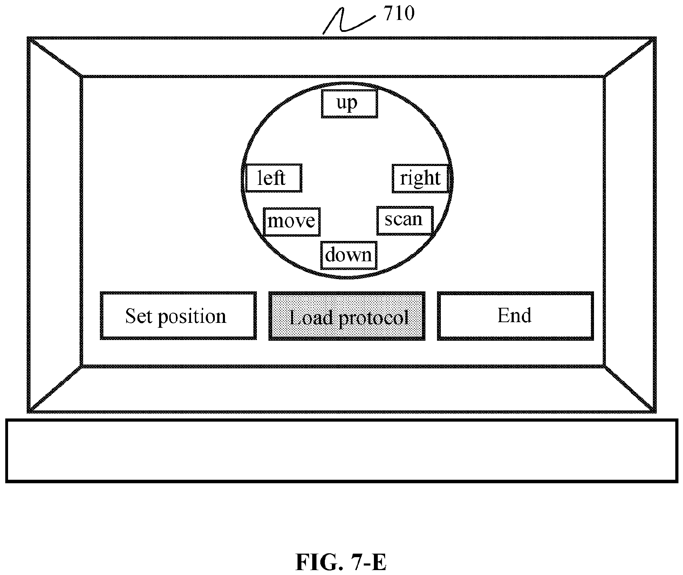

Refer back to Table 1, the third operation may be "loading a scanning protocol". A notification corresponding to the operation "loading a scanning protocol" may be provided, e.g., the "load protocol" button may be lit or flashing indicating a recommended action "pressing the button" (see FIG. 7-E). Simultaneously or successively one or more enable signals corresponding to the operation "loading a scanning protocol" (also the action regarding the operational component "pressing the button") may be generated. Via the correct action, the operation "loading a scanning protocol" may be started. When the operation "loading a scanning protocol" has been started, the corresponding notification may be closed, simultaneously or successively the enable signal(s) may be turned off.

Refer back to Table 1, the fourth operation may be "moving the table to a scanning position." The corresponding operational components may include the buttons "move" in the virtual control box 720 and the physical control box 730. A notification corresponding to the operation "moving the table to a scanning position" may be provided, for example, the buttons "move" in the virtual control box 720 and the physical control box 730 may be lit or flashing indicating a recommended action "pressing the button". Simultaneously or successively one or more enable signals corresponding to the operation "moving the table to a scanning position" (also the action regarding the operational component "pressing the button") may be generated. Via the correct action, the operation "moving a table to a scanning position" may be started. When the operation has been started, the corresponding notification may be closed, simultaneously or successively the enable signal may be turn off.

Refer back to Table 1, the fifth operation may be "starting the scanning." The corresponding operational components may include the buttons "scan" in the virtual control box 720 and the physical control box 730. A notification corresponding to the operation "start the scanning" may be provided, for example, the buttons "scan" in the virtual control box 720 and the physical control box 730 may be lit and flashing indicating a recommended action "pressing the button." Simultaneously or successively one or more enable signals corresponding to the operation "start the scanning" (also the action regarding the operational component "pressing the button") may be generated. Via the correct action, the operation "start the scanning" may be started. When the operation has been started, the corresponding notification may be closed, simultaneously or successively the enable signal(s) may be turned off.

Refer back to Table 1, the last operation may be "ending the scanning." A notification corresponding to the operation "end the scanning" may be provided, e.g., the "end" button may be lit and flashing indicating a recommended action "pressing the button." Simultaneously or successively one or more enable signals corresponding to the operation "ending the scanning" (also the action regarding the operational component "pressing the button") may be generated. When the operation has been started, the corresponding notification may be closed, simultaneously or successively the enable signal(s) may be turned off. As shown in Table 1, after the operation "ending the scanning" has been completed, it may indicate that the workflow has been finished.

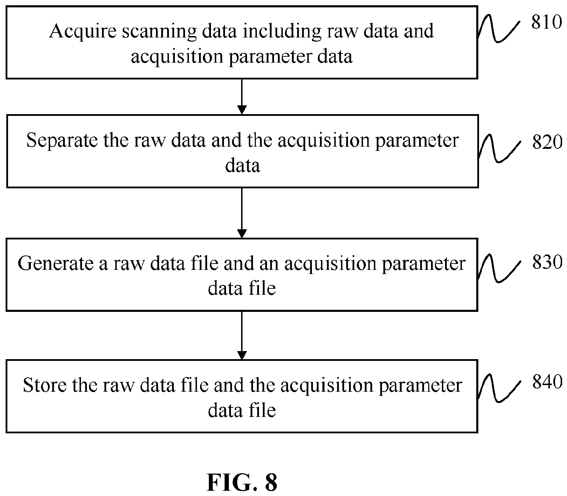

FIG. 8 illustrates an exemplary process for data acquisition and data storage according to some embodiments of the present disclosure. The process may be performed by the data storing/deleting module 320. In step 810, scanning data may be acquired. The data acquisition may be performed by the acquisition module 110. In some embodiments, the scanning data may include raw data (also referred to as "signals") and acquisition parameter data, or the like, or any combination thereof. In some embodiments, the raw data may be acquired in a PET system, a CT system, a SPECT system, an MRI system, or the like, or a combination thereof. The raw data may include radiation data. The radiation may include a particle ray (e.g., positron, neutron, proton, electron, µ-meson, heavy ion, or the like), a photon beam (e.g., γ-ray, α-ray, β-ray, X-ray, ultraviolet, laser, or the like), or the like, or a combination thereof. Merely by way of example, in a PET system, the raw data may include γ-ray related data or signals. In some embodiments, the acquisition parameter data may include information related to a subject (e.g., name, age, gender, height, weight, health history, or the like), environmental information (e.g., temperature, humidity, or the like), system setting data (e.g., power-on setting), a scanning parameter (e.g., a scanning protocol, scanning time, scanning position, scanning intensity, or the like), updating data during a scanning process (e.g., current operation in a scanning process, current scanning position, current position of a table), or the like, or a combination thereof. In some embodiments, the raw data may be corresponding to the acquisition parameter data.

In step 820, the raw data and the acquisition parameter data may be separated based on a separation standard. The separation standard may be based on a default setting of the imaging system 100, or may be determined by an operator (e.g., a doctor). For instance, the raw data and the acquisition parameter data may be separated according to file format, data format, data property, acquisition order, or the like, or a combination thereof. In some embodiments, the separation process may be dynamic or static. For example, during the acquisition process, the raw data and the acquisition parameter data may be acquired separately based on a certain order (e.g., the raw data may be acquired first and the acquisition parameter data later, or vice versa). As another example, the raw data and the acquisition parameter data may be separated after the acquisition process is completed.

In step 830, a raw data file and an acquisition parameter data file may be generated. The formats of the raw data file and the acquisition parameter data file may be the same or different. For example, the raw data file may be a binary file, and the acquisition parameter data file may be a DCM-formatted file that may be checked and modified during the acquisition process. In some embodiments, the raw data file and the acquisition parameter data file may be pre-generated respectively before the acquisition process (step 810), and the generated data files may be used to store the raw data and the acquisition parameter data during the acquisition process. In some embodiments, during the acquisition process, more than one raw data files may be generated. For instance, for different portions of the body of a subject (e.g., the head, the chest, the liver, or the like), different raw data files may be generated. In some embodiments, a public acquisition parameter data file may be used. For instance, during the acquisition process, one or more acquisition parameters may be loaded when needed.

In step 840, the raw data file and the acquisition parameter data file may be stored in the storage module 130 or any storage disclosed anywhere in the present disclosure. Merely by way of example, the storage may include a hard disk, a floppy disk, a cloud storage, a magnetic tape, a compact disk, a removable storage, or the like, or a combination thereof. In some embodiments, different data files may be stored in a same storage position or in different storage positions. For example, the raw data file and the corresponding acquisition parameter data file may be stored in a same folder. As another example, the raw data acquired from different portions of the body of the subject may be stored in different folders. In some embodiments, relevant data files may be stored based on a certain storage rule. As used herein, relevant data files may refer to data files corresponding to a same subject or data files corresponding to a same acquisition process. For example, storage routes of the relevant data files may be generated and provided in a table (e.g., an excel-formatted table). As another example, relevant data files may share a common storage route.

It should be noted that the flowchart described above is provided for the purposes of illustration, not intended to limit the scope of the present disclosure. For persons having ordinary skills in the art, multiple variations and modifications may be reduced to practice in the light of the present disclosure. However, those variations and modifications do not depart from the scope of the present disclosure. For example, step 810 and step 820 may be performed simultaneously or alternately. As another example, the storing the raw data file and the acquisition parameter data file may be performed in real time during the acquisition process.

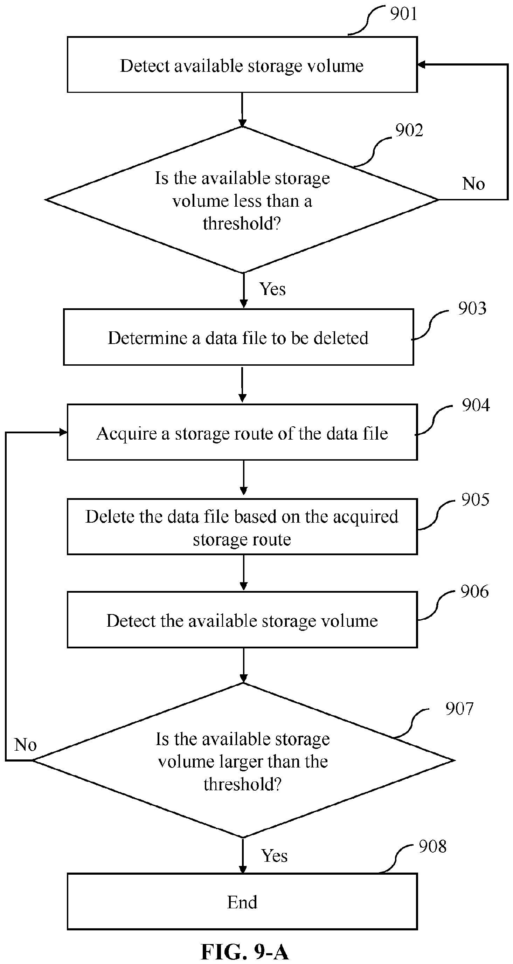

FIG. 9-A is a flowchart illustrating a process for deleting data according to some embodiments of the present disclosure. The process for deleting data may be performed by the data storing/deleting control module 320. The data may be stored in the storage module 130 and/or any storage disclosed anywhere in the present disclosure, or known in the art. In some embodiments, the storage module 130 and/or any storage may include a disk used to store data files and a corresponding database used for analyzing, organizing, or managing the data files. The database may include a plurality of data records. Merely by way of example, a data file (e.g., a file including one or more images of or otherwise relating to a subject) may be stored in the disk; a corresponding data record including the storage route of the data file may be found in the database. As used herein, the storage route may refer to a route that indicates the storage position of the data file. The data file may be identified via the data record.

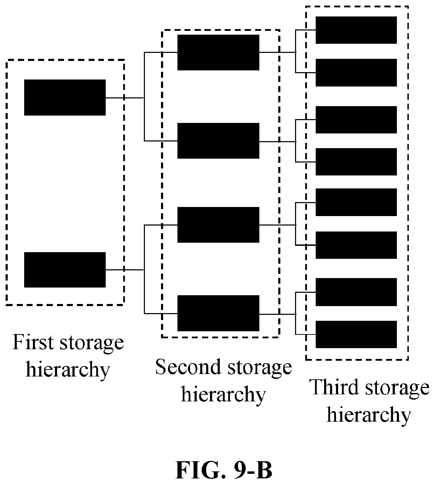

In some embodiments, in a DICOM database, a storage architecture including three storage hierarchies may be used including, for example, a first storage hierarchy, a second storage hierarchy and a third storage hierarchy (see FIG. 9-B). In some embodiments, a data record may include a unique identifier (also referred to "primary key"), and the data record may be uniquely identified by the identifier. In some embodiments, the data record may include a plurality of foreign keys used to establish relationships with other data records. For example, for a data record A, one of the foreign keys may be "ID of subject A," and the "ID of subject A" may relate to several other data records, e.g., a data record whose primary key is "ages of all subjects" and a data record whose primary key is "genders of all subjects." In some embodiments, the three storage hierarchies may be built according to the relationships (see FIG. 9-B).

As shown in FIG. 9-A, in step 901 the process may begin by detecting an available storage volume. The available storage volume may be an available storage volume of the storage module 130 or any storage disclosed anywhere in the present disclosure or known in the art. In some embodiments, the available storage volume may be detected automatically according to a certain time interval (e.g., a day, a week, a month, or the like), based on instructions (e.g., a request for detecting after a scanning is completed) by an operator (e.g., a doctor), or triggered by a triggering event (e.g., before or when a scanning is initiated, before or when one or more data files are to be stored, before or when the system is in the operation mode for a predefined period of time, or the like).

In step 902, the process may determine whether the available storage volume is less than a threshold. If the answer is "yes," the process may proceed to step 903. Alternatively, if the answer is "no," the process may return to step 901 to start a new process, or the process may end. In some embodiments, the threshold may be a default setting of the imaging system 100, or may be set by an operator (e.g., a doctor) according to some characteristics (e.g., usage frequency of the system, number of data files to be stored, or the like).

In step 903, a data file to be deleted (or referred to as a "spare data file") may be determined. As used herein, a data file to be deleted or a spare data file may refer to a data file that may satisfy a preset condition, e.g., difference between storage time of the data file and current time is larger than a preset threshold (e.g., three months), the data file includes uncompleted data (e.g., uncompleted scanning signals), or the like.

In step 904, a storage route of the spare data file may be acquired. As used herein, a storage route may refer to a route that indicates a storage position of a data file. For example, the storage route of a data file that is stored in a disk may be expressed as "E:/Storage/MR data/." In some embodiments, the storage route of the spare data file may be determined by searching a corresponding data record in the database. As used herein, a corresponding data record may refer to a data record that corresponds to a spare data file.

In some embodiments, a data record may include a plurality of fields. As described above, in a DICOM database, three storage hierarchies may be generated. In some embodiments, as shown in FIG. 9-B, a first field may be set as a first search keyword, and a first plurality of data records including the first field may be designate to belong to the first storage hierarchy. In some embodiments, the identifiers of the first plurality of data records may be set as foreign keys on the basis of which the second storage hierarchy may be generated. Then a second field may be set as a second search keyword, and a second plurality of data records including the second field may be designated to belong to the second storage hierarchy. In some embodiments, the identifiers of the second plurality of data records may be set as foreign keys on the basis of which the third storage hierarchy may be generated. Then the data records in the third storage hierarchy may be selected as the corresponding data records. Storage routes of the spare data files may be obtained from the corresponding data records. In some embodiments, the second plurality of data records may be ranked according to when a data record is created, last revised, last viewed or otherwise accessed, or stored (or referred to storage time), and the data record with the earliest storage time may be selected. Similarly then the identifier of the data record with the earliest storage time may be set as a foreign key on the basis of which the third storage hierarchy may be determined. The data records in the third storage hierarchy may be selected as the corresponding data records. Storage routes of the spare data files may be obtained from the corresponding data records.

Merely by way of example, a first field "examination completed" may be set as a first search keyword, and a first plurality of data records may be designate to belong to the first storage hierarchy. The identifiers of the first plurality of data records may be set as foreign keys and the second storage hierarchy may be generated. A second field "unprotected" may be set as a second search keyword, and a second plurality of data records may be designate to belong to the second storage hierarchy. The second plurality of data records may be ranked according to when a data record is created, last revised, last viewed or otherwise accessed, or stored (or referred to storage time), and the data record with the earliest storage time may be selected. Then the identifier of the data record with the earliest storage time may be set as a foreign key and the third storage hierarchy may be generated. The data record(s) in the third storage hierarchy may be selected and the storage route of the spare data file may be obtained from the corresponding data record.

In step 905, the spare data file identified in step 903 may be deleted based on the acquired storage route. In some embodiments, the data file may be deleted from the system, and information relating to the data file (including e.g., filename, size, storage date, deleting date, or the like) may be provided to the operator (e.g., a doctor). In some embodiments, a notification requesting user instructions for confirming deletion and/or keeping a backup copy of the spare data file may be provided. In some embodiments, the spare data file may be removed from the system (e.g., the storage module 130) and transmitted to a secondary storage device (e.g., a hard disk for backup) for backup.

In step 906, after the identified spare data file is deleted, the process may detect the available storage volume. In step 907, the process may determine whether the available storage volume exceeds the threshold. If the answer is "no," the process may return to step 904 to identify more data files to be deleted until the available storage volume exceeds the threshold. If the answer is "yes," in step 908, the process may end.

FIG. 10 is a flowchart illustrating a process for scanning according to some embodiments of the present disclosure. The process for scanning may be performed by the acquisition module 110. As shown in FIG. 10, in step 1001, the process may initiate a scanning protocol. As used herein, the scanning protocol may include one or more parameters relating to the scanning, e.g., the number of table positions of the table, an order of the table positions, the number of scanning positions, a scanning sequence of the scanning positions, the start time and the end time of acquiring signals at each scanning position, etc. The scanning protocol may be a default setting of the imaging system 100, or may be set by an operator (e.g., a doctor) under different situations (e.g., different health conditions may correspond to different scanning requirements).

In step 1002, a table may be moved to a scanning position based on the scanning protocol. The moving of the table may be performed or coordinated by the operation control module 310. In step 1003, a signal may be acquired at the scanning position. The acquired signal may be stored in the acquisition module 110, the storage module 130, or any storage disclosed anywhere in the present disclosure. In some embodiments, the acquired signal may be further transmitted to the reconstruction module 410 for image reconstruction. In some embodiments, information about the scanning position may be recorded in step 1003. Exemplary information about the scanning position may include scanning states relevant to the scanning position (e.g., a state indicative of the scanning has not been performed, a state indicative of the scanning is being performed, a state indicative of the scanning is completed, a state indicative of the scanning is interrupted, or the like), the amount of the acquired signals, etc.

In step 1004, a determination may be made as to whether the scanning is completed. If the answer is "yes," (i.e., it is determined that the scanning is completed), the process may return to step 1001 to start a new process, or the process may end. If the answer is "no," (i.e., it is determined that the scanning is not completed), the process may proceed to step 1005 to determine whether an interruption of the positions of the tables occurs. When an interruption occurs, the scanning position at which the scanning is being performed may be recorded as an interrupted scanning position. If the answer is "no," (i.e., it is determined that the scanning is not interrupted), the process may return to step 1002 to move the table to another scanning position based on the scanning protocol to continue the scanning. If the answer is "yes," (i.e., it is determined that the scanning is interrupted), the process may proceed to step 1006. In step 1006, a determination is made as to whether a supplemental scanning may be performed. As used herein, a supplemental scanning may refer to a scanning performed at the scanning position where an interruption occurs (also refer to as "the interrupted scanning position"). A supplemental scanning may be performed according to the scanning protocol (e.g., signals acquired from all scanning positions are needed for image reconstruction), and/or a user input (e.g., a user determines to perform a supplemental scanning). If in step 1006 the answer is "no," (i.e., it is determined that no supplemental scanning is to be performed), the process may return to step 1001 to start a new process, or the process may end. If the answer is "yes," (i.e., it is determined that a supplemental scanning is to be performed), the process may proceed to step 1007.

In step 1007, incomplete signal(s) acquired from the scanning of the subject corresponding to the interruption may be deleted. The incomplete signal(s) may be deleted by the data storing/deleting control module 320. In some embodiments, the incomplete signal(s) may be stored in a data file and the data file may be linked with a data record. For example, the data record may include a unique identifier (e.g., "data regarding interrupted scanning"). The data storing/deleting control module 320 may identify the data record and delete the incomplete signal(s) if needed (more details regarding the data deleting may be found in FIG. 9). In some embodiments, the operation control module 310 may include a data storing/deleting unit (not shown) used to delete the incomplete signal(s).

In step 1008, the scanning protocol may be updated and the supplemental scanning may be started. In some embodiments, the scanning protocol may be updated by updating the scanning sequence, the start time and/or the end time of acquiring signals at the remaining scanning positions, the states of the scanning positions (e.g., a state indicative of completed scanning, a state indicative of interrupted scanning), and/or any other information relating to the supplemental scanning. To start the supplemental scanning, the process may return to step 1002 to move the table to the interrupted scanning position, and signals may be acquired from a scanning at the interrupted scanning position.

It should be noted that the flowchart described above is provided for the purposes of illustration, not intended to limit the scope of the present disclosure. For persons having ordinary skills in the art, multiple variations and modifications may be reduced to practice in the light of the present disclosure. However, those variations and modifications do not depart from the scope of the present disclosure. For example, step 1004 may be unnecessary, or step 1004 may be performed between any two steps of the process. As another example, a storing step or a caching step may be added between any two steps, in which signals or intermediate data may be stored or cached.

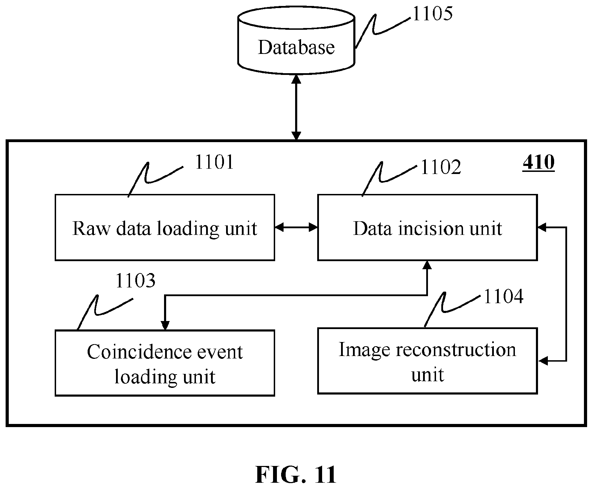

FIG. 11 is a block diagram illustrating an architecture of the reconstruction module 410 according to some embodiments of the present disclosure. As shown in FIG. 11, the reconstruction module 410 may include a raw data loading unit 1101, a data incision unit 1102, a coincidence event loading unit 1103, an image reconstruction unit 1104, and/or other units not shown in the module according to some embodiments of the present disclosure. In some embodiments, the reconstruction module 410 may be connected or otherwise communicate with a database 1105. The database 1105 may be integrated in the storage module 130 or the acquisition module 110, or any storage disclosed anywhere in the present disclosure. The raw data loading unit 1101, the data incision unit 1102, the coincidence event loading unit 1103, the image reconstruction unit 1104, and the database 1105 may be connected with each other via a wired connection (e.g., a metal cable, an optical cable, a hybrid cable, or the like, or any combination thereof) or a wireless connection (e.g., a Local Area Network (LAN), a Wide Area Network (WAN), a Bluetooth, a ZigBee, a Near Field Communication (NFC), or the like, or any combination thereof).

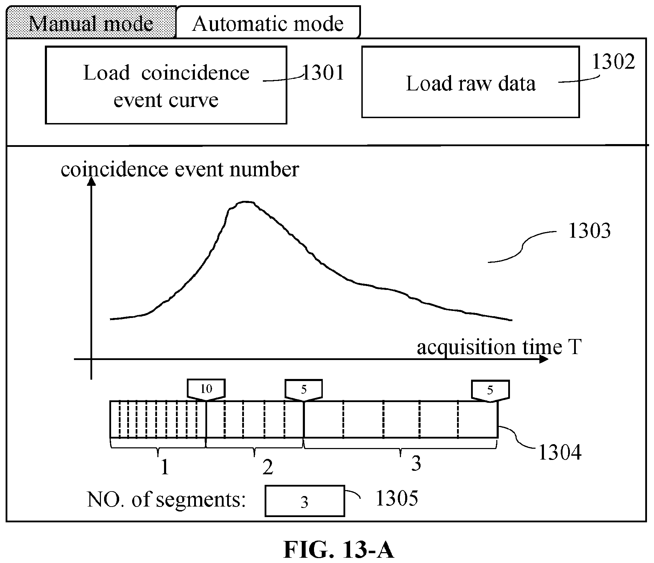

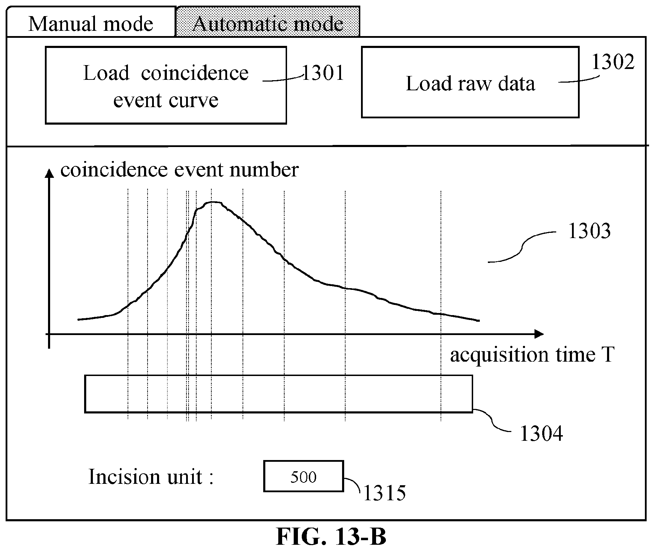

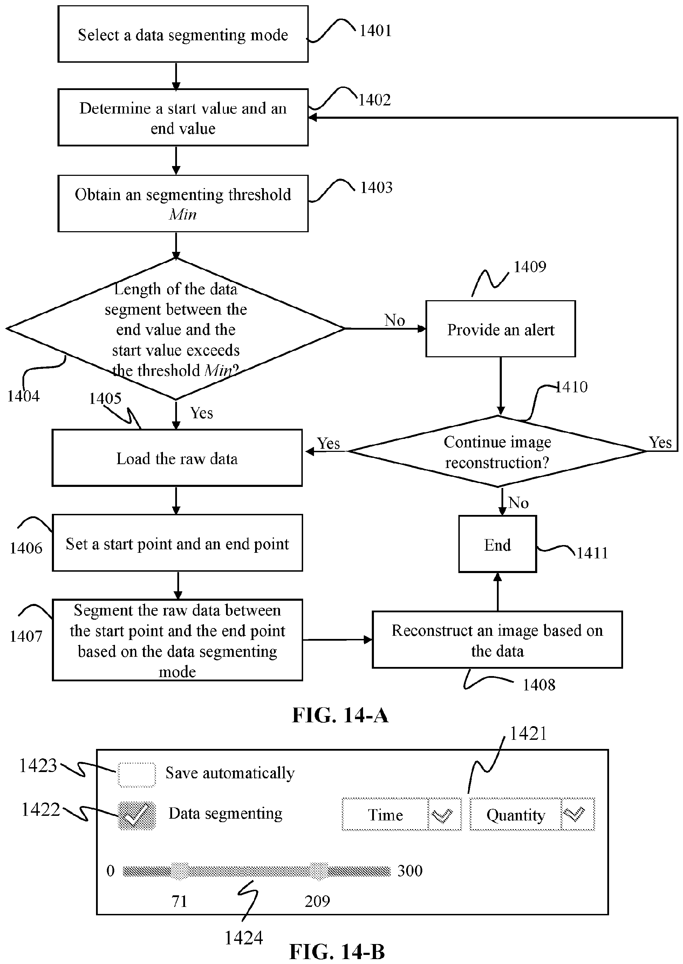

The raw data loading unit 1101 may load raw data from the storage module 130 or the acquisition module 110, or any storage disclosed anywhere in the present disclosure or known in the art. In some embodiments, a raw data slider may be generated (see FIGs. 13-A, 13-B or 14-B). The loaded raw data may be transmitted to the data incision unit 1102 to be further incised. As used herein, the term "incise" may also be referred to as "segment." In some embodiments, one or more parameters regarding the raw data may be displayed on the raw data slider. The parameters may include acquisition time, acquisition quantity, or the like.

The coincidence event loading unit 1103 may load data related to coincidence events from a storage, e.g., any storage mentioned above. In some embodiments, a coincidence event curve may be generated (see FIGs. 13-A and 13-B). In some embodiments, the coincidence event curve may be generated based on the data relating to the coincidence events acquired within a certain time interval or within the whole acquisition process.

The data incision unit 1102 may incise (also referred to as "segment") the raw data. In some embodiments, the data incision unit 1102 may receive the raw data and the data relating to the coincidence events (e.g., the coincidence event curve). In some embodiments, the data incision unit 1102 may incise the raw data based on an incision mode (also referred to as "segmenting mode"). The incision mode may include a time-based mode, a quantity-based mode, or the like. In some embodiments, the data incision unit 1102 may incise the raw data based on an incision mode. The incision mode may include a manual mode, an automatic mode, or the like. In some embodiments, the raw data may be incised based on the coincidence event curve. For example, the raw data may be incised into a plurality of segments according to the slope of the coincidence event curve. In some embodiments, the data incise unit 1102 may include an analysis unit (not shown) used to determine whether wrong data or incomplete data is received. If wrong data or incomplete data is loaded from the raw data loading unit 1101 or the coincidence event loading unit 1103, a feedback may be provided.

The image reconstruction unit 1104 may reconstruct an image (e.g., a PET image, a CT image, a PET/CT image, an MR image, or the like, or a combination thereof) based on the incised data. The reconstructed image may be stored in the storage module 130 or any storage disclosed anywhere in the present disclosure or known in the art. In some embodiments, the reconstructed image or intermediate data generated during the reconstruction process may be transmitted to the incision unit 1102, and may be used as a reference parameter for incising the raw data.

The database 1105 may be used to organize or manage the raw data or the data relating to the coincidence events. In some embodiments, the database 1105 may be integrated in the storage module 130, and may provide storage routes of the raw data and the data related to the coincidence events. For example, the raw data loading unit 1101 and the coincidence event loading unit 1103 may search and load the raw data and the data related to the coincidence events from the storage module 130 via the database 1105. In some embodiments, the database 1105 may include one or more sub-databases (not shown). The sub-databases may organize or manage the raw data and the data related to the coincidence events respectively.

It should be noted that the description above is provided for the purposes of illustration, not intended to limit the scope of the present disclosure. For persons having ordinary skills in the art, multiple variations and modifications may be reduced to practice in the light of the present disclosure. However, those variations and modifications do not depart from the scope of the present disclosure. For example, a storage device (not shown) used to store the raw data and the data related to the coincidence events may be integrated in the reconstruction module 410. As another example, the raw data loading unit 1101 and the coincidence event loading unit 1103 may be integrated in an independent unit configured for loading both the raw data and the data related to the coincidence events.

FIG. 12 illustrates an exemplary process for image reconstruction according to some embodiments of the present disclosure. In step 1201, the raw data may be loaded. The data loading may be performed by the raw data loading unit 1101. In some embodiments, the raw data may be loaded from the acquisition module 110, the storage module 130, or any storage disclosed anywhere in the present disclosure or known in the art. The loaded raw data may be processed (e.g., a data slider may be generated based on the raw data, see FIG.13-A or FIG. 13-B). In step 1202, the data relating to coincidence events (e.g., acquisition time, the number of coincidence events, arrival time, time of flight, angle, intensity, etc.) may be loaded. The data may be loaded by the coincidence event loading unit 1103. Similarly, the data relating to coincidence events may be loaded from any storage mentioned above. In some embodiments, the loaded data relating to coincidence events may be processed, for example, a coincidence event curve (e.g., see FIG.13-A or FIG.13-B) may be generated. In some embodiments, the coincidence event curve may be loaded directly from any storage mentioned above. As shown in FIG. 13-A or FIG. 13-B, the horizontal axis may represent the acquisition time T, and the vertical axis may represent the number of coincidence events. It may be seen that the number of the coincidence events may vary with the acquisition time T.

In step 1203, a data incision mode may be selected. The data incision mode may include a manual mode and an automatic mode. In the manual mode (see details in FIG. 13-A), the raw data may be incised into a plurality of segments based on the absolute value of the slope of the coincidence event curve. Each segment may include a plurality of frames. In some embodiments, one or more parameters may be set by a user or an operator (e.g., a doctor). The parameters may include the number of segments, the number of frames in each segment, or the like, or a combination thereof. The sizes of the plurality of segments may be the same as or different from each other. The numbers of the frames in each segment may be the same as or different from each other. In the automatic mode (see details in FIG. 13-B), the raw data may be incised into a plurality of frames according to the incision unit automatically. As used herein, the incision unit (also referred to as "segmenting unit") may refer to that the raw data may be incised based on the variation of the number of the coincidence events. For example, if the incision unit is set as 500, it means that the raw data may be incised into a plurality of frames, each frame may correspond to 500 coincidence events.

In step 1204, if the manual mode is selected, the raw data may be incised manually. In some embodiments, the manual incision may indicate that one or more incision parameters including, for example, the number of segments, the size of (or the number of frames in) a segment, the number of coincidence events in a frame, etc., may be provided by an operator. As shown in FIG. 13-A, an interface may be shown. The interface may include a plurality of sections, including a coincidence event curve loading section 1301, a raw data loading section 1302, a coincidence event curve 1303, a raw data slider 1304, an input box 1305, or the like. One or more parameters may be set via the interface. For example, the number of segments may be set, e.g., 3 in the input box 1305. See the data slider 1304, the raw data may be incised into three segments including segment 1, segment 2 and segment 3. The sizes of the segments may be set based on the absolute value of the slope of the coincidence event curve. As is known, each segment may include a plurality of frames, and each frame may include a plurality of raw data used for image reconstruction. The numbers of frames in each segment may be different according to quality requirements during image reconstruction. For example, the segment 1 may include ten frames, the segment 2 may include five frames, and the segment 3 may include five frames. The quality of the reconstructed image may be influenced by the number of segments and/or frames in each segment.

In step 1205, if the automatic mode is selected, the raw data may be incised automatically according to the coincidence event curve. For example, a specific section corresponding to a specific number of coincidence events on the coincidence event curve may correspond to a frame on the data slider. As shown in FIG. 13-B, in the automatic mode, an incision unit may be set, e.g., 500 via an input box 1315. As used herein, the incision unit may refer to that the raw data may be incised based on the variation of the number of the coincidence events. As is known, the number of the coincidence events may vary with the acquisition time. For example, if the incision unit is set as 500, the raw data may be incised into a plurality of frames, each frame may correspond to 500 coincidence events. In some embodiments, under the automatic mode, incision parameters may be selected based on the coincidence event curve. For instance, the incision unit corresponding to where the slope of the coincidence event curve is steep is smaller than the incision unit corresponding to where the slope of the coincidence event curve is relatively flat. A steep slope of the coincidence event curve may indicate that the number of the coincidence events may vary rapidly with the acquisition time. A relatively flat slope of the coincidence event curve may indicate that the number of the coincidence events may vary slowly with the acquisition time. As used herein, a frame formed by way of incising a segment according to a small incision unit has fewer coincident events in the frame than a frame formed by way of incision according to a large incision unit.

In step 1206, one or more images may be reconstructed based on the frames of each segment incised in step 1204 or 1205. The image reconstruction may be performed by the image reconstruction unit 1104. The reconstructed image may include a PET image, a CT image, a PET/CT image, an MR image, or the like, or a combination thereof.

It should be noted that the description above is provided for the purposes of illustration, not intended to limit the scope of the present disclosure. For persons having ordinary skills in the art, multiple variations and modifications may be reduced to practice in the light of the present disclosure. However, those variations and modifications do not depart from the scope of the present disclosure. For example, step 1204 and step 1205 may be performed simultaneously or successively. As another example, a storing step may be added. The incised raw data may be stored and further processed if needed.

FIG. 14-A illustrates an exemplary process for image reconstruction according to some embodiments of the present disclosure. In step 1401, a data incision mode (also referred to as "segmenting mode") may be selected. The selection may be performed by the data incision unit 1102. The data incision mode may include a time-based mode and a quantity-based mode. In the time-based mode, the raw data may be incised according to the acquisition time, e.g., a segment of the raw data within a time interval may be incised. In the quantity-based mode, the raw data may be incised according to the acquisition quantity, e.g., a specific quantity of raw data may be incised. In some embodiments, a raw data slider according to the acquisition time or the quantity, corresponding to the time-based mode or the quantity-based mode, may be provided as illustrated in FIG. 14-B.

In step 1402, a start value and an end value according to acquisition time or acquisition quantity relating to the raw data to be processed may be determined. The raw data may be generated in one or a series of scanning. The start value and/or the end value may be set based on a default setting of the imaging system 100, or may be set by an operator (e.g., a doctor) according to image quality requirements for reconstruction (e.g., spatial resolution, definition, signal to noise ratio, contrast, or the like, or a combination thereof). For instance, if the time-based mode is selected in step 1401, the difference between the start value and the end value may correspond to an acquisition time interval. As is known, the image quality may be directly influenced by the raw data. For example, if a segment incised from the raw data is too small (i.e., the acquisition time interval is too small), the spatial resolution of the final reconstructed image may be little due to incomplete information regarding the coincidence events in the segment. As another example, if a segment incised from the raw data is too large (i.e., the acquisition time interval is too large), noises or irrelevant information in the final reconstructed image may be increased.

In step 1403, an incision threshold *Min* may be set. The incision threshold *Min* may be a default setting of the imaging system 100, or may be set by an operator (e.g., a doctor) under different situations. For example, the threshold *Min* may be a minimum incision size that the system may perform. As another example, the threshold *Min* may be determined based on image quality requirements during reconstruction (e.g., a specific contrast of the reconstructed image may correspond to a minimum incision size).

In step 1404, the length of the data segment between the end value and the start value may be calculated, and compared with the threshold *Min* to determine whether the length exceeds the threshold *Min.* If the answer is "yes," (i.e. the length exceeds the threshold *Min*), the raw data may be loaded in step 1405. In some embodiments, the raw data may be loaded from the acquisition module 110, the storage module 130, or any storage disclosed anywhere in the present disclosure or known in the art. In some embodiments, a raw data slider may be provided based on the raw data. In some embodiments, the data slider may be generated dynamically in an on-line mode or may be generated in an off-line mode. As used herein, an on-line mode may refer to that the image reconstruction may be performed during the acquisition process. The off-line mode may refer to that the image reconstruction may be performed after the acquisition process is completed.

In step 1406, a start point and an end point may be set on the data slider as illustrated in FIG. 14-B. As illustrated in FIG. 14-B, the data slider may take the form of a double Vernier, a scale, two input boxes (not shown), a table, or the like, or a combination thereof. Merely by way of example, the start point and the end point may be set by sliding the double Vernier or inputting values in the input boxes. In some embodiments, if the length *N* of the data slider is larger than the threshold *Min*, the start point may be set within the range (0, *N-Min*)*.*

In step 1407, a data segment between the start point and end point may be incised based on the data incision mode. For example, in the time-based mode, the data segment during a certain time range may be incised; in the quantity-based mode, the data segment during a certain quantity range may be incised. In step 1408, one or more images may be reconstructed based on the data segment. After the images are reconstructed, the process may end in step 1411, or may return step 1401 to start a new process.

If in step 1404 the answer is "no," (i.e. the length does not exceed the threshold *Min*), an alert may be provided in step 1409. For instance, the alert may be expressed as "the length to be incised is too small, continue?". In some embodiments, a notification may be provided. For instance, the notification may indicate that the final reconstructed image may be of low quality due to the small incision length. In step 1410, a determination may be made as to whether to continue an image reconstruction. If the answer is "no," (i.e. the image reconstruction is not to be performed), the process may end in step 1411. If the answer is "yes," (i.e. the image reconstruction is to be performed), the process may return to step 1402 to determine a new start value and a new end value. In some embodiments, the process may proceed to step 1405 and the reconstruction process may still continue. As is known, if the length of the data segment between the end value and the start value is smaller than the threshold *Min*, the image reconstruction also can be performed but the quality of the final reconstructed image may be low.

It should be noted that the above description is provided for the purposes of illustration, not intended to limit the scope of the present disclosure. For persons having ordinary skills in the art, multiple variations and modifications may be reduced to practice in the light of the present disclosure. However, those variations and modifications do not depart from the scope of the present disclosure. For example, in step 1402, it is unnecessary to determine a start value and an end value, merely a length of data segment may be determined. As another example, step 1405 may be performed at first, i.e., the raw data may be loaded at first. As a further example, the start point may be the same with the start value or not, similarly the end point is.

FIG. 14-B illustrates an exemplary interface according to some embodiments of the present disclosure. As illustrated in FIG. 14-B, the interface may include a plurality of sections, including 1421, 1422, 1423, and 1424. In section 1421, it may be seen that two icons (e.g., time and quantity) may be used for selecting an incision mode (the time-based incision mode or the quantity-based mode). In section 1422, it may indicate that a data incision is running if the box is checked. In section 1423, if the box is checked, it may indicate the related information (including the data slider, the start value, the end value, or the like) may be saved automatically in the acquisition module 110, the storage module 130, or any storage disclosed anywhere in the present disclosure or known in the art. A data slider is illustrated in section 1424. The data slider may take the form of the double Vernier, the scale, or the like, or a combination thereof.

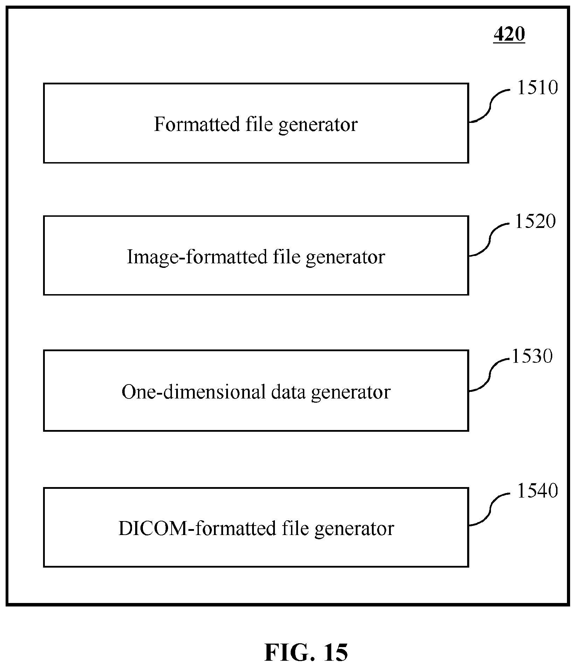

FIG. 15 is a block diagram illustrating an architecture of the report generation module 420 according to some embodiments of the present disclosure. The report generation module 420 may be configured to generate a report based on information about a subject. The report may include basic information (e.g., age, gender, weight, height, or the like), one or more parameters relating to a health examination (e.g., a scanning parameter used in a health examination, etc.), health related information (e.g., image(s), health tips, diagnosis, or the like), or the like, or a combination thereof. The format of the report may include video, audio, text, picture, or the like, or a combination thereof. In some embodiments, the report may be arranged in a report file. The report file may include a plurality of report sections (e.g., a report section used to shown basic information, a report section used to show PET images, a report section used to show CT images, or the like, or a combination thereof). Merely by way of example, the report file may be a DICOM-formatted file, the report file may be linked to a data record that is identified by a unique identifier (more details regarding the data record and/or the identifier may be found in FIG. 9).

As shown in FIG. 15, the report generation module 420 may include a formatted file generator 1510, an image-formatted file generator 1520, a one-dimensional data generator 1530, a DICOM-formatted file generator 1540, and/or any other components for implementing various functions in accordance with the present disclosure.

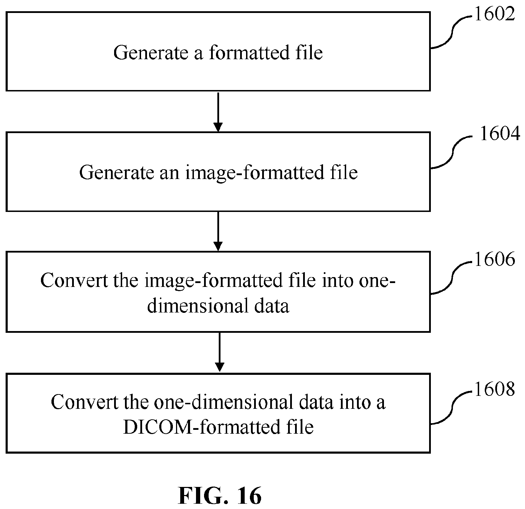

The formatted file generator 1510 may be used to generate a formatted file. In some embodiments, the formatted file may include a plurality of sections. The sections may include a basic information section, an examination section, a health related information section, or the like, or a combination thereof. In some embodiments, the formatted file may be generated from a template file by filling corresponding contents to different sections of the template file (see details in FIG. 16). The formatted file or the template file may be a Hyper Text Markup Language (HTML) format file, an Active Server Pages (ASP) format file, a Hypertext Preprocessor (PHP) format file, or the like, or a combination thereof.

The image-formatted file generator 1520 may be used to generate an image-formatted file. The image-formatted file may be generated by converting a formatted file into one or more images. The image may be generated by creating a screenshot of a part of the formatted file. As used herein, a part may refer to a section, several sections, a part of a section, or the whole file. Merely by way of example, a first image may be generated by creating a screenshot of a first part including a PET image; a second image may be generated by creating a screenshot of a second part including a CT image, or the like. In some embodiments, the image may be a Red Green Blue (RGB) image, e.g., a Bitmap (BMP) format image, a Portable Network Graphic (PNG) format image, a Joint Picture Group (JPG) format image, or the like, or a combination thereof. The one or more images in the image-formatted file may be arranged in a particular order according to a parameter (e.g., names of the corresponding parts).

The one-dimensional data generator 1530 converts the image-formatted file into one-dimensional data. In some embodiments, the one-dimensional data may be generated from one or more RGB images in the image file. In such embodiments, a RGB image may be converted into a grayscale image according to a mapping relationship. The mapping relationship may refer to mapping a color of the RGB image to a grayscale value of the grayscale image. The mapping relationship may be generated by an image processing software (e.g., Matlab, or the like). Then the grayscale image is converted into one-dimensional data.

The DICOM-formatted file generator 1540 may be used to generate a DICOM-formatted file based on the one-dimensional data. The one-dimensional data may be written into the imaging system 100 in the DICOM format. In some embodiments, the DICOM-formatted file may include a plurality of report sections (e.g., a report section used to shown basic information, a report section used to show PET images, a report section used to show CT images, or the like, or a combination thereof).

It should be noted that the description above is provided for the purposes of illustration, not intended to limit the scope of the present disclosure. Any one of the components may be divided into two or more sub-components. As another example, the components may be partially integrated in one or more independent components or share one or more units. As a further example, one or more of the components may be implemented via a computing device (e.g., a desktop, a laptop, a mobile phone, a tablet, a wearable computing device, or the like).

FIG. 16 is a flowchart illustrating a process for generating a report according to some embodiments of the present disclosure. As shown in FIG. 16, in step 1602, the process may begin by generating a formatted file. The formatted file may be generated by the formatted file generator 1510. As described in connection with FIG. 15, the formatted file may be generated based on a template file. The template file may include a plurality of sections, e.g., a basic information section in which basic information regarding a subject (e.g., age, gender, weight, height, or the like) may be added to, an examination section in which parameters relating to scanning or reconstruction (e.g., scanning protocol, scanning time, reconstruction sequence, or the like) may be added to, and a health related information section in which images and/or diagnosis may be added to, or the like. In some embodiments, the template file may be editable. For example, one or more sections of the template file may be added, deleted, and/or modified based on a default setting of the imaging system 100 or user instructions. In some embodiments, the template file may be multi-lingual, e.g., English, Spanish, French, Japanese, Chinese, or the like, or a combination thereof. In some embodiments, the template file may be a HTML format file that may be run by a HTML browser (e.g., Internet Explorer, Firefox, or the like). For instance, a HTML format file may be generated by filling corresponding contents into the sections of the HTML format template via the HTML browser. In some embodiments, the section of the template file may be identified via an index. A formatted file may be generated by filling corresponding contents into the corresponding section according to the index. For example, an index may be used to identify a section of the template file for PET images, and the PET images may be added into the section according to the index.

In step 1604, an image-formatted file may be generated. The image-formatted file may be generated by the image-formatted file generator 1520. In some embodiments, the image-formatted file may be generated by converting the formatted file into one or more images. The image may be generated by creating a screenshot of a part in the formatted file (see details in FIG. 15). For example, for a HTML format file, screenshots may be generated by a HTML browser (e.g., Internet Explorer, Firefox, or the like).

In step 1606, the image-formatted file is converted into one-dimensional data. The converting may be performed by the one-dimensional data generator 1530. In some embodiments, the one-dimensional data may be generated from one or more RGB images in the image-formatted file. A RGB image may be converted into a grayscale image according to a mapping relationship. The mapping relationship may refer to mapping a color of the RGB image with a grayscale value of the grayscale image. In some embodiments, the grayscale values of the grayscale image may vary within, for example, 0 to 250 bites.

In step 1608, the one-dimensional data is converted into a DICOM-formatted file. The conversion may be performed by the DICOM-formatted file generator 1540. The one-dimensional data is written into the DICOM-formatted file. The DICOM-formatted file may be further stored in the storage module 130, or any storage disclosed in the present disclosure or know in the art. In some embodiments, the DICOM-formatted file may be linked with a corresponding data record with a unique identifier (see details in FIG. 9).

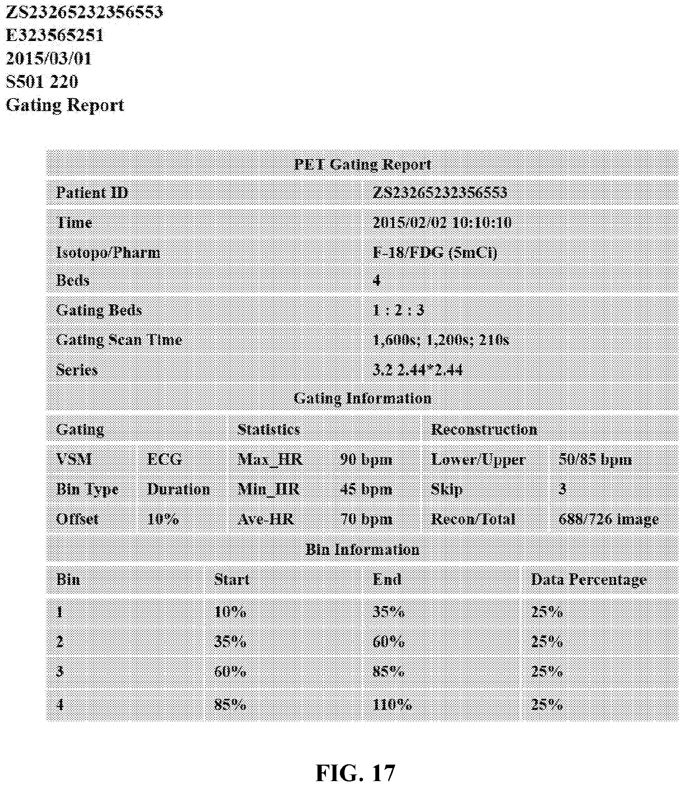

FIG. 17 illustrates an example of a gating report according to some embodiments of the present disclosure. A gating report may be generated by analyzing results of a scanning (e.g., a PET scanning) and results of a reference test (e.g., an electrocardiogram test (ECG)) together. As used herein, the reference test may refer to a test about physiological activities of a subject. Merely by way of example, the physiological activities may be electrical activity of the heart, electrical activity of the lung, electrical activity of the brain, or the like, or a combination thereof. As shown in FIG. 17, the gating report may include a plurality of sections, e.g., basic information about a subject (e.g., patient ID), examination related parameters, gating information, bin information, or the like. The examination related parameters may include isotope/ pharmaceutical used in the PET scanning, beds, gating beds, gating scan time, series, or the like. The gating information may include gating (e.g., VSM, bin type, offset), statistics (Max HR, Min HR, Ave HR), reconstruction related information (lower/upper, skip, recon/total), or the like. The bin information may include bin, start, end, data percentage, or the like.

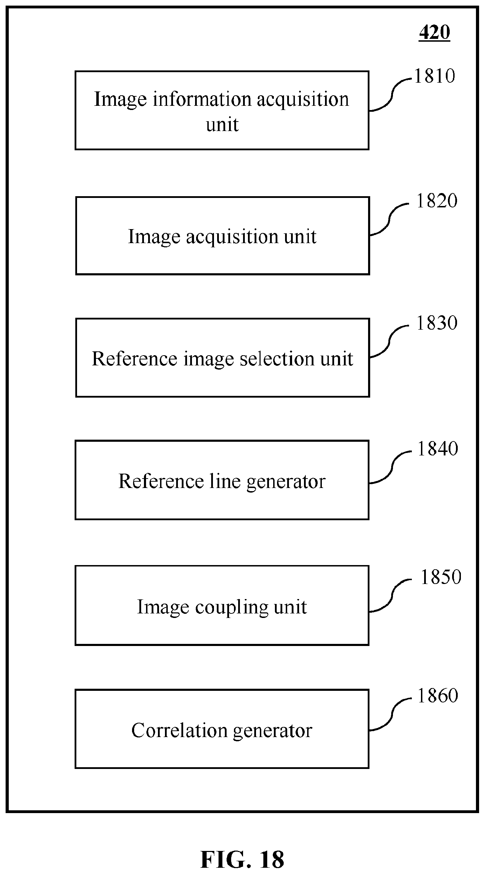

FIG. 18 is a block diagram illustrating an architecture of the report generation module 420 according to some embodiments of the present disclosure. As shown in FIG. 18, the report generation module 420 may include an image information acquisition unit 1810, an image acquisition unit 1820, a reference image selection unit 1830, a reference line generator 1840, an image coupling unit 1850, a correlation generator 1860, and/or any other components for implementing various functions in accordance with the present disclosure.

The image information acquisition unit 1810 may acquire image information about one or more images. The image information may include raw data or signals used to generate the images, and characteristics of the images. The characteristics of the images may include types of the images (e.g., PET, CT, MRI, SPECT, or the like), image thickness, image color, brightness, contrast, resolution, or the like, or a combination thereof.

The image acquisition unit 1820 may acquire one or more images. The images may include a CT image, a PET image, a SPECT image, a MR image, or the like, or a combination thereof. The images may be acquired from the reconstruction module 410, the storage module 130, or any storage disclosed anywhere in the present disclosure. In some embodiments, the acquired images may include one or more images to be processed (e.g., to be printed) and one or more reference images. In some embodiments, the acquired images may be modified. For example, the imaging system 100 may modify three-dimensional positions of the object presented in the images (also refer to as "a profile of the subject" presented in the image) according to the corresponding raw data or signals. For example, before modification, a trans-axial view of the object may be seen in the image; and after the modification, a coronal view of the object may be seen in the image.

The reference image selection unit 1830 may select a reference image from the acquired images. As used herein, a reference image may refer to an image that may be used as a reference in the processing of one or more images. In some embodiments, a reference line may be selected based on the reference image (more details are described below). In some embodiments, the reference image may be selected based on the image information (e.g., a processing parameter) or according to a user instruction. For example, the system may select a SPECT image in response to a user instruction (e.g., a user instruction received from the interface control unit 530). In some embodiments, the reference image may be an image presenting a trans-axial view, a coronal view, or a sagittal view, or the like. In some embodiments, the reference image may be editable. For example, as described above, the imaging system 100 may modify three-dimensional position of the object presented in the reference image according to the corresponding raw data or signals used to generate the reference image.

The reference line generator 1840 may generate a reference line based on the reference image. As used herein, a reference line may refer to a line used for establishing a correlation among the reference image and the images to be processed. In some embodiments, the reference line may be set in any position of the reference image. In some embodiments, a plurality of reference lines may be set. In some embodiments, there are more than two reference lines. The spacing between pairs of adjacent reference lines may be the same or different. In some embodiments, the reference line may be editable. For example, a reference line may be extended, shortened, moved, or rotated. In some embodiments, the reference line may be set according to a default setting of the imaging system 100. For example, a plurality of candidate reference lines may be provided by the imaging system 100, and one or more reference lines may be selected if needed.

The image coupling unit 1850 may couple the reference line with the acquired images. In some embodiments, the images may be modified (e.g., may be reconstituted) via a data reconstitution method. The data reconstitution method may include Multi-Planar Reformation (MPR), Maximum Intensity Projection (MIP), or the like, or a combination thereof. For example, the imaging system 100 may modify three-dimensional position of the object presented in the image according to the corresponding raw data or signals used to generate the image. The modified images may be coupled with the reference line. In some embodiments, the objects presented in the modified images may be presented based on a same three-dimensional position of the object.

The correlation generator 1860 may establish a correlation among the images based on the image information, the reference image, and/or the reference line. The correlation among the images may include processing parameters of the reference image and the images to be processed. The processing parameters may include a parameter related to printing an image (e.g., a reference image, a reference line, type setting, or the like), a parameter related to generating a report (e.g., the number and/or positions of images used in the report, spacing between any two images in the report, formats of the images, or the like), or the like, or a combination thereof. The correlation among the images may be generated by analyzing all or partial of the acquired images.

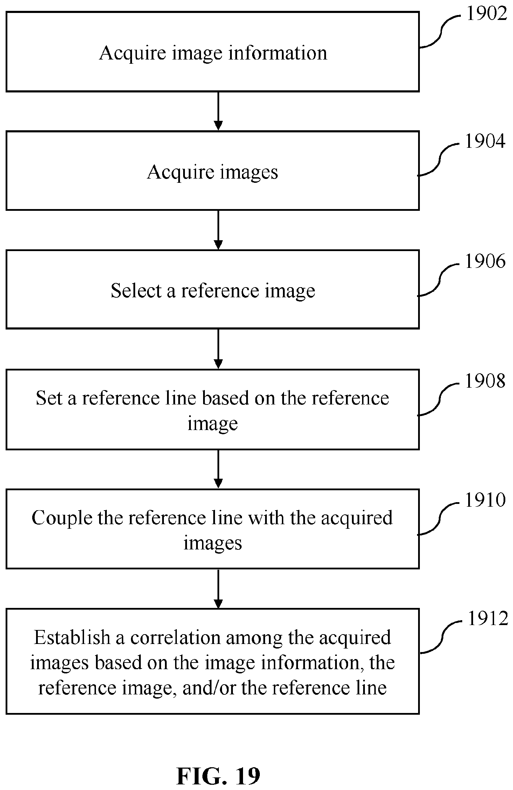

FIG. 19 is a flowchart illustrating a process for generating a report according to some embodiments of the present disclosure. As shown in FIG. 19, in step 1902, the process may begin by acquiring image information. The image information may be acquired by the image information acquisition unit 1810. The image information may include, for example, raw data or signals used to generate the images, characteristics of the images. The characteristics of the images may include, for example, types of the images (e.g., PET, CT, MRI, SPECT, or the like), image thickness, image color, brightness, contrast, resolution, or the like, or a combination thereof. As used herein, an image thickness may refer to the thickness of a region (of a subject) represented in an image.

In step 1904, images may be acquired. The images may be acquired by the image acquisition unit 1820. The images may be acquired from the reconstruction module 410, the storage module 130, or any storage disclosed anywhere in the present disclosure. In some embodiments, the images may include one or more images to be processed (e.g., to be printed) and one or more reference images.

In step 1906, a reference image may be selected. The reference image may be selected by the reference image selection unit 1830. As described in connection with FIG. 18, the reference image may be selected based on the image information or a user instruction. In some embodiments, the reference image may be an image acquired by scanning the whole body of the subject, or may be an image acquired by scanning a portion (e.g., the thorax) of the body of the subject along a direction (e.g., trans-axial direction, sagittal direction, coronal direction, or the like).

In step 1908, a reference line may be set based on the reference image. The reference line may be set by the reference line generator 1840. As described in connection with FIG. 18, the reference line may be set manually by an operator (e.g., a doctor). The reference line may be editable. For instance, the reference line may be extended, shortened, moved, rotated, etc.

In step 1910, the reference line may be coupled with the acquired images including the images to be processed and the reference image. The coupling may be performed by the image coupling unit 1850. In some embodiments, as described in connection with FIG. 18, the images may be modified according to the reference line. The coupling of a reference line with an image may be achieved by marking the image with the reference line.

In step 1912, a correlation among the acquired images may be determined based on the image information, the reference image, and/or the reference line. As described in connection with FIG. 18, the correlation among the images may include processing parameters of the reference image and the images to be processed. The processing parameters may include, for example, a parameter relating to printing an image (e.g., a reference image, a reference line, type setting, or the like), a parameter related to generating a report (e.g., the number and/or positions of images used in the report, formats of the images, or the like), or the like, or a combination thereof. The correlation among the acquired images may be established by the correlation generator 1860. The correlation may be established based on the one or more reference lines coupled with different images. For instance, positions of two different images may be adjusted according to positions of the reference lines presented by the images. In some embodiments, the correlation may be arranged in a report (see details in FIG. 15 or FIG. 16). In some embodiments, the established correlation may be provided to a device, e.g., a display, a terminal (a computer, a mobile phone, or the like), a storage device (e.g., a hard disk, a cloud storage, a removable storage, or the like), a related external device (e.g., a printer), or the like, or a combination thereof. Based on the correlation, the images may be displayed or printed in a partially or completely overlapping manner.

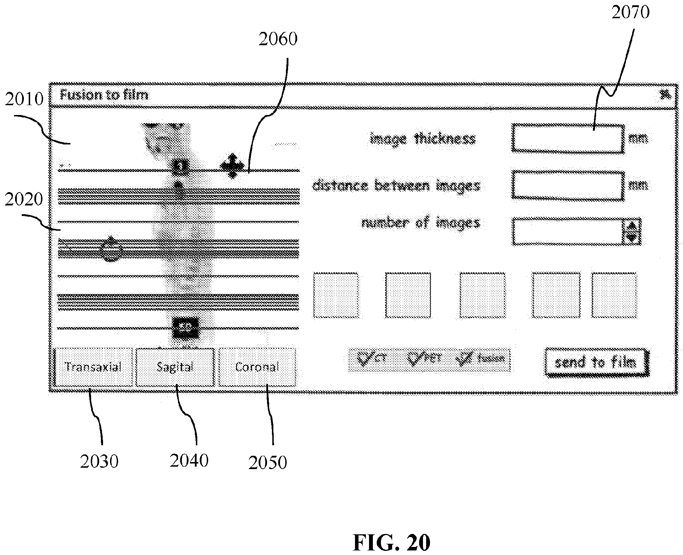

In some embodiments, one or more user interfaces may be provided. An operator (e.g., a doctor) may input some related information via the user interface(s) (see, for example, FIG. 20). For example, the operator may input information about a reference image (e.g., a PET image showing a coronal view of the body of the subject), information about portions of the body of the subject (e.g., size of the head shown in the report), information about the acquired images (e.g., image thickness, positions of the images used in the report, number of images, or the like), information for providing a report (e.g., one or more printing parameters including number of images fused in a film, types of images fused in the film, image thickness, positions of the images used in the report, or the like).

It should be noted that the above description is provided for the purposes of illustration, not intended to limit the scope of the present disclosure. For persons having ordinary skills in the art, multiple variations and modifications may be reduced to practice in the light of the present disclosure. However, those variations and modifications do not depart from the scope of the present disclosure. For example, one or more other optional steps may be added between any two steps of the process illustrated in FIG. 19. Examples of such steps may include storing or caching the acquired information or images. As another example, step 1902 and step 1904 may be integrated into an independent step in which the image information and the images may be acquired simultaneously or successively.

FIG. 20 illustrates an exemplary interface according to some embodiments of the present disclosure. The interface may be provided via the image information acquisition unit 1810. As shown in FIG. 20, the interface may provide a reference image 2010, several candidate reference lines 2020, several reference image selection buttons (e.g., a trans-axial section selection button 2030, a sagittal section selection button 2040, and a coronal section selection button 2050), a reference line selection button 2060, and several input boxes 2070 in which image information (e.g., image thickness, distance between images, number of images, or the like) may be inputted or edited.

A reference image may be selected based on a default setting of the imaging system 100 and/or by an operator (e.g., a doctor) via one or more reference image selection buttons. For example, a coronal section image may be selected as a reference image via the coronal section selection button 2050. The candidate reference lines 2020 may be generated according to one or more scanning parameters, for example, the imaging system 100 may generate several candidate reference lines based on scanning history of a subject. The reference line may be selected via the reference line selection button 2060. In some embodiments, the reference line may be edited via the selection button 2060, e.g., the shape or position of the reference line may be edited. For example, the shape of the reference line may be changed from a linear line to a curve. As another example, the reference line may be moved from a position corresponding to the head to a position corresponding to the liver. One or more parameters may be inputted, e.g., image thickness, distance between images, number of images, or the like. In some embodiments, the images and/or the established correlation may be transmitted to a related device (e.g., a printer) via an icon "send to film."

It should be noted that the above description of the three embodiments are provided for a purpose of comprehending the present disclosure, not intended to limit the scope of the present disclosure. For persons having ordinary skills in the art, various variations and modification may be conducted in the light of the present disclosure. However, the variations and the modifications do not depart from the scope of the present disclosure.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure.

Moreover, certain terminology has been used to describe embodiments of the present disclosure. For example, the terms "one embodiment," "an embodiment," and/or "some embodiments" mean that a particular feature, structure or characteristic described in connection with the embodiment is included in at least one embodiment of the present disclosure. Therefore, it is emphasized and should be appreciated that two or more references to "an embodiment," "one embodiment," or "an alternative embodiment," in various portions of this specification are not necessarily all referring to the same embodiment. Furthermore, the particular features, structures or characteristics may be combined as suitable in one or more embodiments of the present disclosure.

Further, it will be appreciated by one skilled in the art, aspects of the present disclosure may be illustrated and described herein in any of a number of patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as a "block," "module," "engine," "unit," "component," or "system." Furthermore, aspects of the present disclosure may take the form of a computer program product embodied in one or more computer readable media having computer readable program code embodied thereon.

A computer readable signal medium may include a propagated data signal with computer readable program code embodied therein, for example, in baseband or as part of a carrier wave. Such a propagated signal may take any of a variety of forms, including electro-magnetic, optical, or the like, or any suitable combination thereof. A computer readable signal medium may be any computer readable medium that is not a computer readable storage medium and that may communicate, propagate, or transport a program for use by or in connection with an instruction execution system, apparatus, or device. Program code embodied on a computer readable signal medium may be transmitted using any appropriate medium, including wireless, wireline, optical fiber cable, RF, or the like, or any suitable combination of the foregoing.

Computer program code for carrying out operations for aspects of the present disclosure may be written in any combination of one or more programming languages, including an object oriented programming language such as Java, Scala, Smalltalk, Eiffel, JADE, Emerald, C++, C#, VB. NET, Python or the like, conventional procedural programming languages, such as the "C" programming language, Visual Basic, Fortran 2003, Perl, COBOL 2002, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Although the above disclosure discusses through various examples what is currently considered to be a variety of useful embodiments of the disclosure, it is to be understood that such detail is solely for that purpose, and that the appended claims are not limited to the disclosed embodiments. For example, although the implementation of various components described above may be embodied in a hardware device, it may also be implemented as a software only solution-e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. Rather, embodiments lie in less than all features of a single foregoing disclosed embodiment.

In some embodiments, the numbers expressing quantities of ingredients, properties such as molecular weight, reaction conditions, and so forth, used to describe and claim certain embodiments of the patent are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameters set forth in the written description and are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the patent are approximations, the numerical values set forth in the specific examples are reported as precisely as practicable.

In closing, it is to be understood that the embodiments of the patent disclosed herein are illustrative of the principles of the embodiments of the patent.

## Claims

1. A method for imaging, the method comprising:
providing an imaging device having a table;
performing, based on a scanning protocol, scans of a subject located on the table at multiple table positions, each scan covering a portion of the subject;
acquiring data based on the scans of the subject;
reconstructing an image based on the acquired data;
generating a formatted file;
generating an image-formatted file using a screenshotting method;
converting the image-formatted file into a one-dimensional data set; and
converting the one-dimensional data set into a DICOM-formatted file.

2. The method of claim 1, the generating a formatted file comprising:
generating a template, the template comprising at least a section identified by an index;
obtaining information regarding the subject; and
adding the information into the section according to the index.

3. The method of claim 1 or 2, the generating an image-formatted file comprising:
obtaining a color image-formatted file,
wherein the converting the image-formatted file into a one-dimensional data set comprises
mapping the color image-formatted file with a grayscale image-formatted file; and
converting the grayscale image-formatted file to the one-dimensional data set.

4. The method of any one of claims 1-3, the performing scans of a subject comprising:
detecting an interruption of the table positions according to the scanning protocol;
deleting data acquired from the scan of the subject corresponding to the interruption;
updating the scanning protocol; and
performing a supplemental scanning from the interrupted table position based on the updated scanning protocol.

5. The method of any one of claims 1-4, the scanning protocol comprising the number of the table positions and an order of the table positions.

6. The method of claim 4 or 5, the deleting data acquired from the scan of the subject corresponding to the interruption comprising:
detecting a status of each one of the table positions;
determining the interrupted table position based on the status of the table positions; and
deleting the data acquired from the interrupted table position.

7. The method of any one of claims 4-6, the performing a supplemental scanning from the interrupted table position based on the updated scanning protocol comprising:
marking the interrupted table position; and
performing the supplemental scanning from the marked table position.

8. The method of any one of claims 1-7, further comprising segmenting the acquired data based on a segmenting mode.

9. The method of claim 8, the segmenting mode comprising a time-based mode or a quantity-based mode.

10. The method of claim 9, wherein in the time-based mode, the acquired data is segmented based on acquisition time.

11. The method of claim 9 or 10, wherein in the quantity-based mode, the acquired data is segmented based on acquisition quantity of the acquired data.

12. The method of any one of claims 8-11, the segmenting the acquired data comprising:
segmenting the data based on a coincidence event curve; and
generating a data section based on the segmented data,
wherein the reconstructing an image is based on the data section.

13. The method of any one of claims 8-11, the segmenting the acquired data comprising:
setting a threshold;
setting a start value and an end value of the acquired data;
calculating a difference between the start value and the end value; and
providing an alert when the difference is less than the threshold.

14. An imaging system (100), comprising:
an imaging device having a table (160);
an operation control module (310) configured to perform, based on a scanning protocol, scans of a subject located on the table at multiple table positions, each scan covering a portion of the subject;
an acquisition module (110) configured to acquire data based on the scans of the subject; and comprising a processing engine (140) including
a reconstruction module (410) configured to reconstruct an image based on the acquired data; and the said processing engine (140) including a report generating module (420), the report generation module (420) being adapted to
generate a formatted file,
generate an image-formatted file using a screenshotting method,
convert the image-formatted file into a one-dimensional data set, and
convert the one-dimensional data set into a DICOM-formatted file.

15. The imaging system (100) of claim 14, further comprising a data deleting module,
the operation control module (310) configured to detect an interruption of the table positions according to the scanning protocol;
the data deleting module configured to delete data acquired from the scans of the subject corresponding to the interruption;
the operation control module (310) configured to update the scanning protocol, and perform a supplemental scanning from the interrupted table position based on the updated scanning protocol.

## Patentansprüche

1. Verfahren zur Bildgebung, wobei das Verfahren umfasst:
Bereitstellen einer Bildgebungsvorrichtung mit einem Tisch;
Durchführen, auf Basis eines Scanprotokolls, von Scans an einem auf dem Tisch befindlichen Subjekt bei mehreren Tischstellungen, wobei jeder Scan einen Abschnitt des Subjekts abdeckt;
Erfassen von Daten auf Basis der Scans des Subjekts;
Rekonstruieren eines Bildes auf Basis der erfassten Daten;
Erzeugen einer formatierten Datei;
Erzeugen einer Bildformat-Datei unter Verwendung eines Screenshot-Verfahrens;
Umwandeln der Bildformat-Datei in einen eindimensionalen Datensatz; und
Umwandeln des eindimensionalen Datensatzes in eine DICOM-formatierte Datei.

2. Verfahren nach Anspruch 1, wobei das Erzeugen einer formatierten Datei umfasst:
Erzeugen einer Schablone, wobei die Schablone mindestens einen mit einem Index identifizierten Ausschnitt umfasst;
Erhalten von Informationen hinsichtlich des Subjekts; und
Einfügen der Informationen in den Ausschnitt gemäß dem Index.

3. Verfahren nach Anspruch 1 oder 2, wobei das Erzeugen einer Bildformat-Datei umfasst:
Erhalten einer Farbbildformat-Datei,
wobei das Umwandeln der Bildformat-Datei in einen eindimensionalen Datensatz umfasst
Mapping der Farbbildformat-Datei mit einer Graustufenbildformat-Datei; und
Umwandeln der Graustufenbildformat-Datei in den eindimensionalen Datensatz.

4. Verfahren nach einem der Ansprüche 1-3, wobei das Durchführen von Scans eines Subjekts umfasst:
Erkennen einer Unterbrechung der Tischstellungen gemäß dem Scanprotokoll;
Löschen von aus dem Scan des Subjekts erfassten Daten entsprechende der Unterbrechung;
Aktualisieren des Scanprotokolls; und
Durchführung eines zusätzlichen Scans in der unterbrochenen Tischstellung auf Basis des aktualisierten Scanprotokolls.

5. Verfahren nach einem der Ansprüche 1-4, wobei das Scanprotokoll die Anzahl der Tischstellungen und eine Reihenfolge der Tischstellungen umfasst.

6. Verfahren nach einem der Ansprüche 4 oder 5, wobei das Löschen von aus dem Scannen des Subjekts erfassten Daten entsprechend der Unterbrechung umfasst:
Erkennen eines Status jeder einzelnen der Tischstellungen;
Bestimmen der unterbrochenen Tischstellung auf Basis des Status der Tischstellungen; und
Löschen der in der unterbrochenen Tischstellung erfassten Daten.

7. Verfahren nach einem der Ansprüche 4-6, wobei die Durchführung eines zusätzlichen Scans in der unterbrochenen Tischstellung auf Basis des aktualisierten Scanprotokolls umfasst:
Markieren der unterbrochenen Tischstellung; und
Durchführung des zusätzlichen Scans in der markierten Tischstellung.

8. Verfahren nach einem der Ansprüche 1-7 ferner umfassend die Unterteilung der erfassten Daten auf Basis eines Unterteilungsmodus.

9. Verfahren nach Anspruch 8, wobei der Unterteilungsmodus einen zeitbasierten Modus oder einen mengenbasierten Modus umfasst.

10. Verfahren nach Anspruch 9, wobei in dem zeitbasierten Modus die erfassten Daten auf Basis der Erfassungszeit unterteilt werden.

11. Verfahren nach Anspruch 9 oder 10, wobei in dem mengenbasierten Modus die erfassten Daten auf Basis der Erfassungsmenge der erfassten Daten unterteilt werden.

12. Verfahren nach einem der Ansprüche 8-11, wobei die Unterteilung der erfassten Daten umfasst:
Unterteilen der Daten auf Basis einer Koinzidenzereigniskurve; und
Erzeugen eines Datenabschnitts auf Basis der unterteilten Daten,
wobei die Rekonstruktion eines Bildes auf dem Datenausschnitt basiert.

13. Verfahren nach einem der Ansprüche 8-11, wobei die Unterteilung der erfassten Daten umfasst:
Festlegen eines Schwellenwerts;
Festlegen eines Startwertes und eines Endwertes der erfassten Daten;
Berechnen einer Differenz zwischen dem Startwert und dem Endwert; und
Bereitstellen eines Alarms, wenn die Differenz geringer ist als der Schwellenwert.

14. Bildgebungssystem (100), umfassend:
einen Bildgebungsvorrichtung mit einem Tisch (160);
ein Betriebssteuermodul (310), das konfiguriert ist, um auf Basis eines Scanprotokolls Scans eines auf dem Tisch befindlichen Subjekts in mehreren Tischstellungen durchzuführen, wobei jeder Scan einen Abschnitt des Subjekts abdeckt;
ein Erfassungsmodul (110), das konfiguriert ist, um Daten auf Basis der Scans des Subjekts zu erfassen; und umfassend eine Verarbeitungsmaschine (140), einschließlich
eines Rekonstruktionsmodul (410), das konfiguriert ist, um ein Bild auf Basis den erfassten Daten zu rekonstruieren; und wobei die Verarbeitungsmaschine (140) ein Berichtserzeugungsmodul (420) einschließt, wobei das Berichtserzeugungsmodul (420) dazu ausgelegt ist
eine formatierte Datei zu erzeugen,
eine Bildformat-Datei unter Verwendung eines Screenshot-Verfahrens zu erzeugen, die Bildformat-Datei in einen eindimensionalen Datensatz umzuwandeln,
und
den eindimensionalen Datensatz in eine DICOM-Formatdatei umzuwandeln.

15. Bildgebungssystem (100) nach Anspruch 14 ferner umfassend ein Datenlöschmodul,
wobei das Betriebskontrollmodul (310) konfiguriert ist, um eine Unterbrechung der Tischstellungen gemäß dem Scanprotokoll zu erkennen;
das Datenlöschmodul konfiguriert ist, um Daten, die von den Scans des Subjekts erfasst wurden, entsprechend der Unterbrechung zu löschen;
das Betriebssteuermodul (310) konfiguriert ist, um das Scanprotokoll zu aktualisieren und einen zusätzlichen Scan in der unterbrochenen Tischstellung auf Basis des aktualisierten Scanprotokolls durchzuführen.

## Revendications

1. Procédé d'imagerie, le procédé comprenant les étapes consistant à :
prévoir un dispositif d'imagerie qui possède une table ;
effectuer, sur la base d'un protocole de balayage, des balayages d'un sujet situé sur la table dans plusieurs positions de table, chaque balayage couvrant une partie du sujet ;
acquérir des données sur la base des balayages du sujet ;
reconstruire une image sur la base des données acquises ;
générer un fichier formaté ;
générer un fichier au format d'image à l'aide d'un procédé de capture d'écran ;
convertir le fichier au format d'image en un ensemble de données unidimensionnel ; et
convertir l'ensemble de données unidimensionnel en un fichier au format DICOM.

2. Procédé selon la revendication 1, la génération d'un fichier formaté comprenant :
la génération d'un modèle, le modèle comprenant au moins une section identifiée par un index ;
l'obtention d'informations relatives au sujet ; et
l'ajout des informations à la section selon l'index.

3. Procédé selon la revendication 1 ou 2, la génération d'un fichier au format d'image comprenant :
l'obtention d'un fichier au format d'image en couleur,
dans lequel la conversion du fichier au format d'image en un ensemble de données unidimensionnel comprend
le mappage du fichier au format d'image en couleur avec un fichier au format d'image à nuances de gris ; et
la conversion du fichier au format d'image à nuances de gris en l'ensemble de données unidimensionnel.

4. Procédé selon l'une quelconque des revendications 1 à 3, l'exécution des balayages d'un sujet comprenant :
la détection d'une interruption des positions de table selon le protocole de balayage ;
la suppression des données acquises du balayage du sujet correspondant à l'interruption ;
la mise à jour du protocole de balayage ; et
l'exécution d'un balayage supplémentaire à partir de la position de table interrompue sur la base du protocole de balayage mis à jour.

5. Procédé selon l'une quelconque des revendications 1 à 4, le protocole de balayage comprenant le nombre de positions de table et un ordre des positions de table.

6. Procédé selon la revendication 4 ou 5, la suppression des données acquises à partir du balayage du sujet correspondant à l'interruption comprenant :
la détection d'un statut de chacune des positions de table ;
la détermination de la position de table interrompue sur la base du statut des positions de table ; et
la suppression des données acquises à partir de la position de table interrompue.

7. Procédé selon l'une quelconque des revendications 4 à 6, l'exécution d'un balayage supplémentaire à partir de la position de table interrompue sur la base du protocole de balayage mis à jour comprenant :
le marquage de la position de table interrompue ; et
l'exécution du balayage supplémentaire à partir de la position de table marquée.

8. Procédé selon l'une quelconque des revendications 1 à 7, comprenant en outre la segmentation des données acquises sur la base d'un mode de segmentation.

9. Procédé selon la revendication 8, le mode de segmentation comprenant un mode basé sur la durée ou un mode basé sur la quantité.

10. Procédé selon la revendication 9, dans lequel, dans le mode basé sur la durée, les données acquises sont segmentées sur la base de la durée d'acquisition.

11. Procédé selon la revendication 9 ou 10, dans lequel, dans le mode basé sur la quantité, les données acquises sont segmentées sur la base de la quantité d'acquisition des données acquises.

12. Procédé selon l'une quelconque des revendications 8 à 11, la segmentation des données acquises comprenant :
la segmentation des données sur la base d'une courbe d'événement de coïncidence ; et
la génération d'une section de données sur la base des données segmentées,
dans lequel la reconstruction d'une image repose sur la section de données.

13. Procédé selon l'une quelconque des revendications 8 à 11, la segmentation des données acquises comprenant :
la définition d'un seuil ;
la définition d'une valeur de début et d'une valeur de fin des données acquises ;
le calcul d'une différence entre la valeur de début et la valeur de fin ; et
le déclenchement d'une alerte lorsque la différence est inférieure au seuil.

14. Système d'imagerie (100), comprenant :
un dispositif d'imagerie ayant une table (160) ;
un module de commande (310) configuré pour exécuter, sur la base d'un protocole de balayage, des balayages d'un sujet situé sur la table dans plusieurs positions de table, chaque balayage couvrant une partie du sujet ;
un module d'acquisition (110) configuré pour acquérir des données sur la base des balayages du sujet, et comprenant un moteur de traitement (140) qui comprend
un module de reconstruction (410) configuré pour reconstruire une image sur la base des données acquises ; et ledit moteur de traitement (140) comprenant un module de génération de rapports (420), le module de génération de rapports (420) étant adapté pour
générer un fichier formaté,
générer un fichier au format d'image à l'aide d'un procédé de capture d'écran,
convertir le fichier au format d'image en un ensemble de données unidimensionnel, et
convertir l'ensemble de données unidimensionnel en un fichier au format DICOM.

15. Système d'imagerie (100) selon la revendication 14, comprenant en outre un module de suppression de données,
le module de commande (310) étant configuré pour détecter une interruption des positions de table selon le protocole de balayage ;
le module de suppression de données étant configuré pour supprimer les données acquises à partir des balayages du sujet qui correspondent à l'interruption ;
le module de commande (310) étant configuré pour mettre à jour le protocole de balayage, et exécuter un balayage supplémentaire à partir de la position de table interrompue sur la base du protocole de balayage mis à jour.
